# EUROPEAN PATENT SPECIFICATION

(11) **EP 1 373 262 B1**
(45) Date of publication and mention of the grant of the patent: **12.05.2010**
(21) Application number: 02726168.4
(22) Date of filing: 13.03.2002
(51) Int. Cl.: C07D 409/12

(54) **AZACYCLOALKYL SUBSTITUTED ACETIC ACID DERIVATIVES FOR USE AS MMP INHIBITORS**
AZACYCLOALKYL-SUBSTITUIERTE ESSIGSÄURE-DERIVATE ZUR VERWENDUNG ALS MMP-INHIBITOREN
DERIVES D'ACIDE ACETIQUE SUBSTITUES AZACYCLOALKYLE UTILISÉS COMME INHIBITEURS DE MMP

(30) Priority: 14.03.2001 US 275819 P; 22.03.2001 US 278572 P
(43) Date of publication of application: 02.01.2004
(73) Proprietor: Novartis AG, 4056 Basel (CH); Novartis Pharma GmbH, 1230 Wien (AT)
(72) Inventor: FUJIMOTO, Roger, Aki, Morristown, NJ 07960 (US); MCQUIRE, Leslie, Wighton, Warren, NJ 07059 (US); MONOVICH, Lauren, G., Summit, NJ 07901 (US); NANTERMET, Philippe, Lansdale, PA 19446 (US); PARKER, David, Thomas, Livingston, NJ 07039 (US); ROBINSON, Leslie, Ann, Del Mar, CA 92014 (US); SKILES, Jerry, W., New Providence, NJ 07974 (US); TOMMASI, Ruben, Alberto, Whitehouse Station, NJ 08889 (US)
(74) Representative: Patent- und Rechtsanwaltssozietät Maucher, Börjes & Kollegen
(86) International application number: PCT/EP2002/002808
(87) International publication number: WO 2002/072577

(56) References cited:
- EP-A- 0 895 988
- WO-A-01/70690
- WO-A-01/70691
- WO-A-98/50348
- WO-A-99/06340
- WO-A-99/07675
- PIKUL ET AL.: "Potent and selective carboxylic acid-based inhibitors of matrix metalloproteinases." J. MED. CHEM., vol. 44, no. 16, 2001, pages 2499-2502, XP002205901

## Description

### Summary of the Invention

The present invention relates to novel α-(azacycloalkyl)-substituted biarylsulfonamidoacetic acid derivatives described below, as inhibitors of matrix-degrading metalloproteinases, pharmaceutical compositions comprising said compounds, said compounds for use in inhibiting MMP-13 or in treating inflammatory conditions in a mammal, respectively, and their use in the manufacture of a medicament for inhibiting MMP-13 or for treating inflammatory conditions in a mammal, respectively.

The compounds of the invention inhibit matrix degrading metalloproteinases (MMPs), such as gelatinase, stromelysin and collagenase, in particular, collagenase-3 (MMP-13). Thus, the compounds of the invention inhibit matrix degradation and are particularly useful for the prevention or treatment of matrix degrading metalloproteinase dependent pathological conditions, in particular, collagenase-3 dependent pathological conditions in mammals. Such conditions include malignant and nonmalignant tumors (by inhibiting tumor growth tumor metastasis, tumor progression or invasion and/or tumor angiogenesis), such tumors including breast, lung, bladder, colon, ovarian and skin cancer; inflammatory conditions, e.g., arthritis (rheumatoid and osteoarthritis), septic shock, inflammatory bowel disease, Crohn's disease and the like; also inflammatory demyelinating disorders of the nervous system in which myelin destruction or loss is involved (such as multiple sclerosis), optic neuritis, neuromyelitis optica (Devic's disease), diffuse and transitional sclerosis (Schilder's disease) and acute disseminated encephalomyelitis; also demyelinating peripheral neuropathies such as Landry-Guillain-Barre-Strohl syndrome for motor defects; also tissue ulceration (e.g., epidermal and gastric ulceration), abnormal wound healing, periodental disease and bone disease (e.g., Paget's disease and osteoporosis). Ocular applications include the treatment of ocular inflammation, corneal ulcerations, pterygium, macular degeneration, keratitis, keratoconus, open angle glaucoma, retinopathies, and also their use in conjunction with refractive surgery (laser or incisional) to minimize adverse effects. In addition to inhibition of MMPs, certain compounds of this invention may also inhibit TNF-α converting enzyme (TACE). Other conditions to be treated with the compounds of the invention include bronchial disorders (such as asthma), atherosclerotic conditions (by, e.g., inhibiting rupture of atherosclerotic plaques), as well as acute coronary syndrome, heart attacks (cardiac ischemias), strokes (cerebral ischemias), restenosis after angioplasty, and also vascular ulcerations, ectasia and aneurysms.

The preferred compounds of the invention, particularly those of formula I below wherein R is OH and R₁ is acyl, are potent selective inhibitors of MMP-13 (collagenase-3), and are substantially free of MMP-1 (collagenase-1) inhibition and of TNF-converting enzyme (TACE) inhibition. The selective MMP-13 inhibitors are expected to be substantially free of side effects which have been typically associated with MMP inhibitors, e.g., musculoskeletal effects.

WO 01/70691 and WO 01/76090 specifically describe biphenylyl sulfonamidoacetic acid derivatives which thus structurally differ from the compounds of the present invention.

### Detailed Description of the Invention

The present invention, in a first embodiment, relates to compounds as claimed in claims 1, 2 and 4; pharmaceutical compositions comprising them according to claim 4; as well as to said compounds for use according to claim 5 or claim 6 and the use of said compounds in the manufacture of a medicament according to claim 7 or claim 8, all claims being incorporated into the present description by reference.

The present invention relates particularly to the compound of formula I 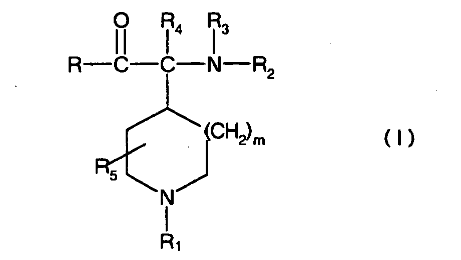
wherein R represents OH ;
R₁ represents
   acyl derived from a carboxylic acid, from a carbonic acid, from a carbamic acid or from a sulfonic acid;
R₂ represents biarylsulfonyl
R₃ represents hydrogen, lower alkyl;
R₄ and R₅ represent hydrogen;
m is 1;
pharmaceutically acceptable prodrug derivatives thereof; pharmaceutically acceptable salts thereof; pharmaceutical compositions comprising said compounds; and their use for inhibiting matrix degrading metalloproteinases and preventing or treating matrix metalloproteinase dependent conditions in mammals.

The compounds of the invention depending on the nature of the substituents, possess one or more asymmetric carbon atoms. Also the cyclohexane substituents are either cis or trans to each other. The resulting diastereoisomers, enantiomers and geometric isomers are encompassed by the instant invention.

Preferred are the compounds of the invention wherein the configuration of the asymmetric carbon atom of the α-amino acid moiety corresponds to that of a D-amino acid precursor and is assigned the (R)-configuration.

Pharmaceutically acceptable prodrug derivatives are those that may be convertible by solvolysis or under physiological conditions to the free acids of the invention and represent carboxylic acids in which the COOH group is derivatized in form of, e.g., an ester derivative, or hydroxamic acids in which the CONHOH group is derivatized, e.g., in form of an optionally substituted O-benzyl derivative.

Prodrug carboxylic acid esters are, e.g., lower alkyl esters, cycloalkyl esters, lower alkenyl esters, benzyl esters, mono or disubstituted lower alkyl esters, e.g., the ω-(amino, mono- or di-lower alkylamino, carboxyl, lower alkoxycarbonyl)-lower alkyl esters, the α-(lower alkanoyloxy, lower alkoxycarbonyl or di-lower alkylaminocarbonyl)-lower alkyl esters, such as the pivaloyloxymethyl ester, and the like conventionally used in the art.

Optionally substituted O-benzyl derivatives of the hydroxamic acids of the invention are, e.g., benzyl or benzyl substituted by lower alkoxy, lower alkyl, halogen, trifluoromethyl and the like.

Pharmaceutically acceptable salts of the acidic compounds of the invention are salts formed with bases, namely, cationic salts such as alkali and alkaline earth metal salts, such as sodium, lithium, potassium, calcium, magnesium, as well as ammonium salts, such as ammonium, trimethyl-ammonium, diethylammonium and tris-(hydroxymethyl)-methylammonium salts.

Similarly, acid addition salts, such as of mineral acids, organic carboxylic and organic sulfonic acids., e.g., hydrochloric acid, methanesulfonic acid, maleic acid, are also possible.

The general definitions used herein have the following meaning within the scope of the present invention or general disclosure, unless otherwise specified.

The term "lower" referred to above and hereinafter in connection with organic radicals or compounds, respectively, defines such as branched or unbranched with up to and including 7, preferably up to and including 4, and advantageously one or two carbon atoms.

A lower alkyl group is branched or unbranched and contains 1 to 7 carbon atoms, preferably 1-4 carbon atoms, and represents, for example, methyl, ethyl, propyl, butyl, isopropyl or isobutyl.

Optionally substituted lower alkyl refers to unsubstituted or substituted straight or branched chain hydrocarbon groups having 1 to 7 carbon atoms.

Substituted lower alkyl refers to lower alkyl groups substituted by one or more of the following groups: halo, trihalomethyl such as CCl₃ or CF₃, hydroxy, alkoxy, alkoxyalkoxy, aryloxy, cycloalkyl, alkanoyl, alkanoyloxy, amino, substituted amino, alkanoylamino, thiol, alkylthio, arylthio, alkylthiono, alkylsulfonyl, arylsulfonyl, aminosulfonyl, nitro, cyano, carboxy, carbamyl, alkoxycarbonyl, aralkoxy or heterocyclyl. Substituted amino refers to amino, mono- or, independently, disubstituted by, e.g., alkyl, aralkyl, cycloalkyl, cycloalkylalkyl, heteroaralkyl, or amino disubstituted by lower alkylene or lower alkylene interrupted by O, S, N-(H, alkyl, acyl or aralkyl) so as to form a heterocyclyl group and the like.

Optionally substituted lower alkoxy (or alkyloxy) group preferably contains 1-4 carbon atoms, and represents, e.g., methoxy, ethoxy, propoxy, isopropoxy, butoxy, isobutoxy, trifluoroethoxy, bis-trifluoroisopropoxy, perfluoroethoxy, trifluoromethoxy or 2-methoxyethoxy.

Halogen (or halo) preferably represents chloro or fluoro but may also be bromo or iodo.

Aryl represents carbocyclic and/or heterocyclic aryl.

Carbocyclic aryl represents monocyclic or bicyclic carbocyclic aryl, for example, phenyl or phenyl mono-, di-, or tri-substituted by radicals selected from optionally substituted lower alkyl, optionally substituted lower alkoxy, lower alkyl-(thio, sulfinyl or sulfonyl), lower alkoxy-lower alkoxy, hydroxy, halogen, cyano, trifluoromethyl, amino, mono- or di-lower alkylamino and heterocycyl; or monosubstituted by lower alkylenedioxy; or 1- or 2-naphthyl.

Monocyclic carbocyclic aryl is preferably phenyl or phenyl mono- or di-substituted by groups as defined above.

Heterocyclic aryl represents monocyclic or bicyclic heteroaryl, for example, pyridyl, quinolinyl, isoquinolinyl, benzothienyl, benzofuranyl, benzopyranyl, benzothiopyranyl, furanyl, pyrrolyl, thiazolyl, isothiazolyl, thiadiazolyl, oxadiazolyl, pyrimidinyl, pyridazinyl, oxazolyl, isoxazolyl, triazolyl, tetrazolyl, pyrazolyl, imidazolyl, thienyl, or any said radical substituted, especially mono- or di-substituted, by, e.g., optionally substituted lower alkyl, optionally substituted lower alkoxy , cyano, hydroxy, acyl, trifluoro-lower alkoxy, trifluoromethyl or halogen. Pyridinyl (pyridyl) represents 2-, 3- or 4-pyridyl, advantageously 3- or 4-pyridyl. Thienyl represents 2- or 3-thienyl, advantageously 2-thienyl. Quinolinyl represents, e.g., 2-, 3- or 4-quinolinyl, advantageously 2-quinolinyl. Isoquinolinyl represents, e.g., 1-, 3- or 4-isoquinolinyl. Benzopyranyl, benzothiopyranyl represent, e.g., 3-benzopyranyl or 3-benzothiopyranyl, respectively. Thiazolyl represents, e.g., 2- or 4-thiazolyl. Triazolyl is 1-, 2- or 5-(1,2,4-triazolyl). Tetrazolyl is 5-tetrazolyl. Imidazolyl is, e.g., 4-imidazolyl. Furanyl represents 2- or 3-furanyl, advantageously 2-furanyl. Isothiazolyl represents, e.g., 3-isothiazolyl. Thiadiazolyl represents, e.g., 2-thiadiazolyl, e.g., 2-[1,3,4]-thiadiazolyl. Oxadiazolyl represents 2-oxadiazolyl, e.g., 2-[1,3,4]-oxadiazolyl. Pyrimidinyl represents, e.g., 2-pyrimidinyl. Pyridazinyl represents, e.g., 3-pyridazinyl.

Biaryl (as in biarylsulfonyl for R₂) represents two covalently linked aryl groups in which each of the aryl groups is either monocyclic carbocyclic aryl or monocyclic heterocyclic aryl. More particularly, biaryl represents monocyclic carbocyclic aryl substituted by 5- or 6-membered heterocyclic aryl, 5- or 6-membered heterocyclic aryl substituted by monocyclic carbocyclic aryl, or 5- or 6-membered heterocyclic aryl substituted by 5- or 6-membered heterocyclic aryl.

Preferred biaryl groups are: (i) phenyl substituted by phenyl which is optionally substituted by radicals selected from Optionally substituted lower alkyl, optionally substituted lower alkoxy, lower alkyl-(thio, sulfinyl or sulfonyl), lower alkoxy-lower alkoxy, hydroxy, halogen, cyano, trifluromethyl, amino, mono- or di-lower alkylamino, heterocyclyl, and lower alkylenedioxy; (ii) thienyl or furanyl substituted by phenyl which is optionally substituted by radicals selected from lower alkyl, lower alkoxy, lower alkyl-(thio, sulfinyl or sulfonyl), lower alkoxy-lower alkoxy, hydroxy, halogen, cyano, trifluromethyl, amino, mono- or di-lower alkylamino, heterocyclyl and lower alkylenedioxy; or (iii) phenyl, thienyl or furanyl substituted by pyridyl which is optionally substituted by lower alkoxy.

In the above biaryl groups phenyl is preferably substituted at the para position, and thienyl and furanyl (e.g., 2- or 3-thienyl and 2- or 3-furanyl) are preferably substituted at the 4- or 5-position.

Particular biaryl groups are, e.g., 4-biphenylyl, 5-phenyl-2-thienyl, 5-pyridyl-2-thienyl, 5-phenyl-2-furanyl, 5-isoxazolyl-2-thienyl, 2-phenyl-5-thiazolyl, 2-phenyl-5-[1,3,4]-thiadiazolyl, 5-thiadiazolyl-2-thienyl, 5-isoxazolyl-2-thienyl and 6-phenyl-3-pyridazinyl, all of which are optionally substituted as indicated herein.

Aryloxyaryl represents preferably phenyl substituted by carbocyclic or heterocyclic aryloxy, preferably monocyclic carbocyclic aryloxy, advantageously at the para position.

Heterocyclyl represents a 5- to 7-membered saturated ring containing one or more heteroatoms selected from N, O and S, e.g., piperidinyl, pyrrolidinyl, morpholinyl, piperazinyl, (N-lower alkyl or aryl-lower-alkyl)-piperazinyl, perhydrofuranyl, perhydropyranyl and the like; also, e.g., tetrahydroisoquinolinyl.

Cycloalkyl-lower alkyl represents, e.g., (cyclopentyl- or cyclohexyl)-(methyl or ethyl).

Acyl represents acyl derived from a carboxylic acid, a carbonic acid, a carbamic add or an organic sulfonic add.

Acyl derived from a carboxylic add represents, e.g., lower alkanoyl, aroyl, aryl-lower alkanoyl, cycloalkylcarbonyl; or lower alkanoyl substituted by, e.g., lower alkoxy, heterocyclyl, cycloalkyl, amino or mono- or di-(lower alkyl or aryl-lower alkyl)-amino, or aryl-lower alkoxy.

Acyl derived from an organic carbonic acid is, e.g., lower alkoxycarbonyl, aryloxycarbonyl, aryl-lower alkoxycarbonyl or cycloalkyloxycarbonyl.

Acyl derived from a carbamic acid represents, for example, aminocarbonyl optionally substituted on nitrogen by one or two substituents selected independently from lower alkyl, cycloalkyl, aryl and aryl-lower alkyl; or heterocylcylcarbonyl in which heterocyclyl represents nitrogen containing heterocyclyl, e.g., piperidino, pyrrolidino, morpholino or 2-tetrahydroisoquinolinyl.

Acyl derived from an organic sulfonic acid represents, for example, lower alkylsufonyl, arylsulfonyl, aryl-lower al kylsulfonyl, cycloalkylsulfonyl or cycloalkyl-lower alkylsulfonyl.

Lower alkanoyl represents, e.g., C₁-C₇-alkanoyl including formyl, and is preferably C₂-C₄-alkanoyl such as acetyl or priopionyl.

Aroyl represents carbocyclic or heterocyclic aroyl, e.g., benzoyl or benzoyl mono- or di-substituted by one or two radicals selected from lower alkyl, lower alkoxy, halogen, cyano and trifluromethyl; or 1- or 2-naphthoyl; and also, e.g., pyridylcarbonyl.

Lower alkoxycarbonyl represents preferably C₁-C₄-alkoxycarbonyl, such as ethoxycarbonyl, propoxycarbonyl, isopropoxycarbonyl, t-butoxycarbonyl and the like.

Lower alkylene represents either straight chain or branched alkylene of 1 to 7 carbon atoms and represents preferably straight chain alkylene of 1 to 4 carbon atoms, e.g., a methylene, ethylene, propylene or butylene chain, or said methylene, ethylene, propylene or butylene chain mono-substituted by C₁-C₃-alkyl (advantageously methyl) or disubstituted on the same or different carbon atoms by C₁-C₃-alkyl (advantageously methyl), the total number of carbon atoms being up to and including 7.

Lower alkylenedioxy is preferably ethylenedioxy or methylenedioxy.

A particular embodiment described in the present disclosure consists of the compounds of formula I in which the asymmetric carbon of the α-amino acid moiety is of the (R)-configuration, namely, compounds of formula II 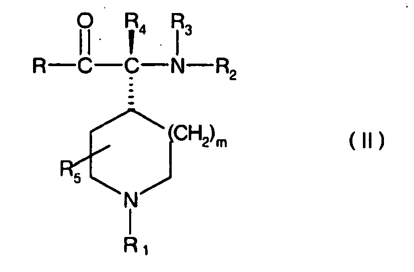
wherein R, R₁-R₅ and m have meaning as defined herein, pharmaceutically acceptable prodrug ester derivatives thereof and pharmaceutically acceptable salts thereof.

Further particular embodiments described in the present disclosure relate to the compounds of formula I or II wherein
(b) R₁ represents acyl derived from a carboxylic acid (amides) (invention embodiment);
(c) R₁ represents acyl derived from a carbonic acid (urethanes) (invention embodiment);
(d) R₁ represents acyl derived from a carbamic acid (ureas) (invention embodiment);
(e) R₁ represents acyl derived from a sulfonic acid (sulfonamides) (invention embodiment);
and pharmaceutically acceptable prodrug ester derivatives thereof; and pharmaceutically acceptable salts thereof.

The invention refers to the claimed compounds which are particularly useful as potent selective inhibitors of MMP-13 (without substantially inhibiting MMP-1 or TACE).

Another particular embodiment of the present disclosure relates to the compounds of the formula I or II wherein R is hydroxy; R₂ represents biarylsulfonyl in which biaryl represents monocyclic carbocyclic aryl substituted by monocyclic carbocyclic or heterocyclic aryl; or biaryl represents 5- or 6-membered heterocyclic aryl substituted by monocyclic carbocyclic or heterocyclic aryl; R₁ represents acyl derived from a carboxylic acid, a carbonic acid, a carbamic acid or a sulfonic acid; R₃ represents hydrogen or lower alkyl; R₄ and R₅ represent hydrogen; m is 1; pharmaceutically acceptable prodrug derivatives thereof; and pharmaceutically acceptable salts thereof.

Preferably described in the present disclosure are the above compounds wherein R₂ represents the radical Ar₂-Ar₁-SO₂- in which Ar₁ is phenylene, furanylene or thienylene; or Ar₁ is thiazolylene, thiadiazolylene or pyridazinylene; and Ar₂ is monocyclic carbocyclic aryl; or Ar₂ is optionally substituted pyridyl; or Ar₂ is optionally substituted isoxazolyl or optionally substituted thiadiazolyl.

Further preferably described in the present disclosure are said compounds wherein Ar₁ is 1,4-phenylene, 2,5-furanylene or 2,5-thienylene; or Ar₁ is 2,5-[1,3,4]-thiadiazolylene or 3,6-pyridazinylene; Ar₂ is monocyclic carbocyclic aryl; or Ar₂ is pyridyl optionally substituted by lower alkyl, lower alkoxy, cyano or trifluoromethoxy; or Ar₂ is 3-isoxazolyl optionally substituted by trifluoromethyly; or Ar₂ is 4-[1,2,3]-thiadiazolyl.

Further preferably described in the present disclosure are the above compounds wherein R₂ represents the radical Ar₂-Ar₁-SO₂- in which Ar₁ is phenylene, furanylene or thienylene; and Ar₂ is monocyclic carbocyclic aryl; or Ar₂ is optionally substituted pyridyl. Further preferred are said compounds wherein Ar₁ is 1,4-phenylene, 2,5-furanylene or 2,5-thienylene; or Ar₂ is monocyclic carbocyclic aryl; or Ar₂ is pyridyl optionally substituted by lower alkyl, lower alkoxy, cyano or trifluoromethoxy.

Another particular embodiment described in the present disclosure is directed to the urethane compound of the formula III 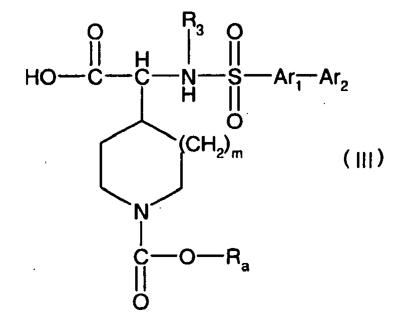
wherein m is one; R₃ represents hydrogen or lower alkyl; Ar₁ represents phenylene, furanylene or thienylene; or Ar₁ represents thiazolylene, thiadiazolylene or pyridazinylene;
Ar₂ represents monocyclic carbocyclic aryl, or optionally substituted pyridyl; or Ar₂ represents optionally substituted isoxazolyl or optionally substituted thiadiazolyl; and Rₐ represents lower alkyl or cycloalkyl; pharmaceutically acceptable prodrug ester derivatives thereof; and pharmaceutically acceptable salts thereof.

Preferably described in the present disclosure are the compounds of formula III wherein m is one; R₃ represents hydrogen or lower alkyl; Ar₁ represents phenylene, furanylene or thienylene; Ar₂ represents monocyclic carbocyclic aryl, or optionally substituted pyridyl); and
Rₐ represents lower alkyl or cycloalkyl; pharmaceutically acceptable prodrug ester derivatives thereof; and pharmaceutically acceptable salts thereof.

Preferred are the (R)-isomers of formula IIIa 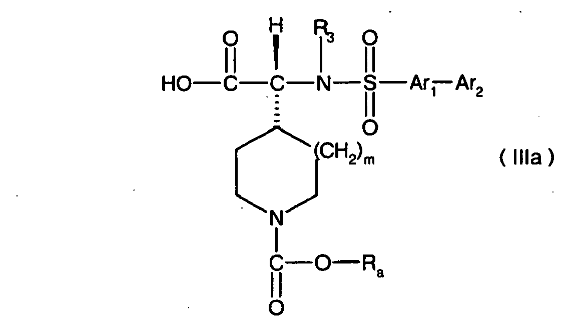 wherein m, R₃, Rₐ, Ar₁ and Ar₂ have meaning as defined above, pharmaceutically acceptable prodrug ester derivatives thereof, and pharmaceutically acceptable salts thereof.

Another particular embodiment described in the present disclosure relates to the amide type compounds of the formula 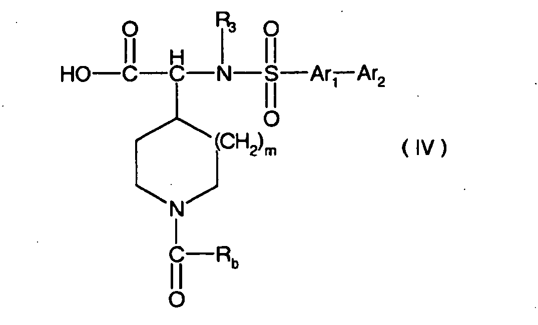 wherein m is one; R₃ represents hydrogen or lower alkyl; Ar₁ represents phenylene, furanylene or thienylene; Ar₂ represents monocyclic carbocyclic aryl or optionally substituted pyridyl; and R_{b} represents lower alkyl, cycloalkyl or aryl; or R_{b} represents lower alkyl substituted by lower alkoxy, cycloalkyl, aryl, heterocyclyl, aryl-lower alkoxy or substituted by amino or (mono- or di-lower alkyl or aryl-lower alkyl)-amino; pharmaceutically acceptable prodrug ester derivatives thereof; and pharmaceutically acceptable salts thereof.

Preferred are the (R)-isomers of formula IVa 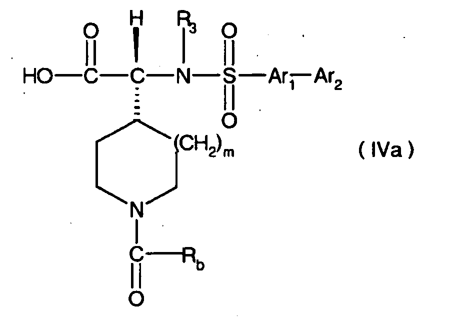 wherein m, R₃, Ar₁, Ar₂ and R_{b} have meaning as defined above; pharmaceutically acceptable prodrug ester derivatives thereof; and pharmaceutically acceptable salts thereof.

A further particular embodiment described in the present disclosure relates to the urea type compounds of formula V 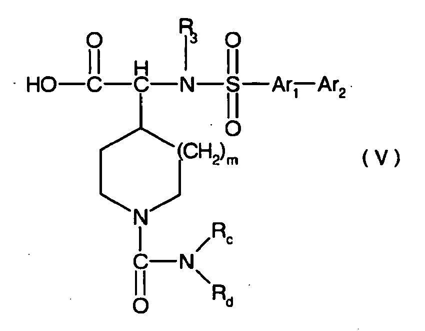 wherein m is one; R₃ represents hydrogen or lower alkyl; Ar₁ represents phenylene, furanylene or thienylene; Ar₂ represents monocarbocyclic aryl or optionally substituted pyridyl; R_{c} and R_{d} represent independently hydrogen, lower alkyl or aryl; or R_{c} and R_{d} together with the nitrogen to which they are attached form a piperidinyl, pyrrolidinyl, morpholinyl, piperazinyl, N-(lower alkyl or aryl-lower alkyl)-piperazinyl or tetrahydroisoquinolinyl ring; pharmaceutically acceptable prodrug ester derivatives thereof; and pharmaceutically acceptable salts thereof.

Preferred are the corresponding (R)-isomers of formula Va 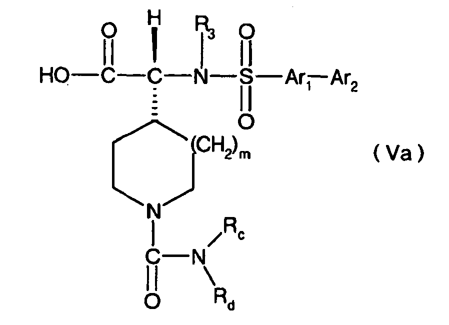 wherein m, R₃, R_{c}, R_{d}, Ar₁ and Ar₂ have meaning as defined above; pharmaceutically acceptable prodrug ester derivatives thereof; and pharmaceutically acceptable salts thereof.

Another particular embodiment described in the present disclosure relates to sulfonyl derivatives of formula VI 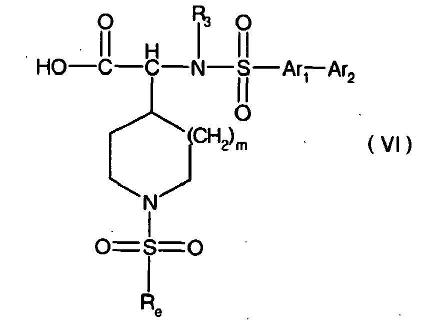 wherein m is one; R₃ represents hydrogen or lower alkyl; Ar₁ represents phenylene, furanylene or thienylene; Ar₂ represents monocyclic carbocyclic aryl or optionally substituted pyridyl; Rₑ represents lower alkyl, cycloalkyl, aryl-lower alkyl or aryl; pharmaceutically acceptable prodrug ester derivatives thereof; and pharmaceutically acceptable salts thereof.

Preferred are the corresponding (R)-isomers of formula VIa 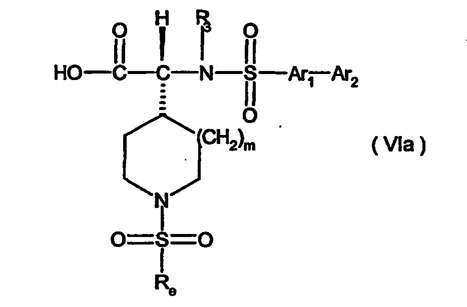 wherein m, R₃, Rₑ, Ar₁ and Ar₂ have meaning as defined above, pharmaceutically acceptable prodrug ester derivatives thereof; and pharmaceutical acceptable salts thereof.

Preferred are the above compounds of formulas III-VI a wherein Ar₁ represents 1,4-phenylene, 2,5-furanylene or 2,5-thienylene; Ar₂ represents phenyl or phenyl substituted by lower alkylenedioxy or phenyl mono- or di-substituted independently by lower alkyl, lower alkoxy, cyano, trifluoromethyl, trifluoromethoxy or halo; pharmaceutically acceptable prodrug ester derivatives thereof; and pharmaceutically acceptable salts thereof.

The compounds of the invention exhibit valuable pharmacological properties in mammals including man, particularly as inhibitors of matrix-degrading matalloproteinase enzymes.

The compounds are therefore particularly useful for the treatment of, e.g., inflammatory conditions such as rheumatoid arthritis, osteoarthritis, of tumors and other metalloproteinase-dependent conditions, e.g., those described hereinabove.

Beneficial effects are evaluated in pharmacological tests generally known in the art, and as illustrated herein.

The above-cited properties are demonstrable in *in vitro and in vivo* tests, using advantageously mammals, e.g., rats, guinea pigs, dogs, rabbits, or isolated organs and tissues, as well as mammalian enzyme preparations. Said compounds can be applied *in vitro* in the form of solutions, e.g., preferably aqueous solutions, and *in vivo* either enterally or parenterally, advantageously orally, e.g., as a suspension or in aqueous solution. The dosage *in vitro* may range between about 10⁻⁵ molar and 10⁻¹⁰ molar concentrations. The dosage *in vivo* may range, depending on the route of administration, between about 0.1 and 100.mg/kg.

Examples of the standard tests are illustrated below.

The activity of test compounds against recombinant human MMP-13 is measured by monitoring the increase in fluorescence intensity of the hydrolysis product Mca-Pro-Leu-Gly COOH resulting from the hydrolysis of Mca-Pro-Leu-Gly-Leu-Dpa-Ala-Arg-CONH₂. The IC₅₀s are estimated by plotting % inhibition of activity versus inhibitor concentration.

The activity of the test compounds against recombinant human MMPs (MMP-1, 3 and 9) is measured using NFF2, a quenched fluorescent peptide substrate. The IC₅₀s are calculated using a 4-parameter logistic fit. The impact of serum on the inhibitory activity of the compounds can also be measured.

The activity of test compounds against recombinant human MMP-7 is measured using a quenched fluorescent substrate FS-6 (Mca-Lys-Pro-Leu-Gly-Leu-Dpa Ala-Arg-NH₂). The IC₅₀s are estimated by plotting % inhibition of activity versus inhibitor concentration.

In addition, the ability of bovine serum albumin to impair the inhibitory potency of the test compound is studied by carrying out the assay in the presence of 1% BSA.

The activity of test compounds against recombinant human MT1-MMP is measured using a fluorogenic peptide substrate, 2-N-methylaminobenzoic acid (Nma)-Gly-Pro-Gln-Gly-Leu-Ala-Gly-Gln-Lys-N^{ε}-(2,4-dinitrophenyl)(Dnp)-NH₂. The reaction is started by the addition of 0.5 nM of enzyme. The inhibition results are expressed as the inhibitor concentration that produces 50% inhibition (IC₅₀) of the activity in the control (non-inhibited reaction).

An *in vitro* assay to determine the ability of test compounds to block cartilage degradation is carried out as follows. Bovine nasal explants are stimulated, in the presence or absence of an inhibitor at various concentrations, for 5 or 6 days with rh-IL-1α and rh-OSM in media. After incubation the media is harvested and replaced with fresh stimuli and inhibitors. On Day 11, the experiment is terminated by harvesting media and digesting the remainder of cartilage with papain. The harvested fluid and pa pain digested cartilage are then subjected to HyPro analysis and the percent inhibition of type II collagen degradation by MMP inhibition is calculated.

*In vivo,* fasted rats are dosed with test compounds and after 4 hours are challenged with an intra-articular injection of rh-tMMP-13. After a further 2 hours the knees are lavaged to collect synovial fluid. The amount of chondroitin sulfate (CS) released into the synovial fluid is measured by ELISA and the % inhibition of CS release calculated.

The inhibition of the production or secretion of TNF-α (by inhibition of TNF-α convertase) can be determined, e.g., as described in Nature, Vol. 370, pp. 555, 558 (1994).

Antiinflammatory activity can be determined in standard inflammation and arthritic animal models well-known in the art, e.g., the adjuvant arthritis model in rats and the collagen II induced arthritis model in mice (Mediators of Inflam., Vol. 1, pp. 273-279 (1992)).

The effect of compounds of the invention on cartilage degradation *in vivo* can be determined in rabbits. Typically, four rabbits are dosed orally with a compound up to four hours before being injected intra-articularly in both knees (N-8) with 40 units of recombinant human stromelysin dissolved in 20 mM tris, 10 mM CaCl₂, and 0.15M NaCl at pH 7.5. Two hours later the rabbits are sacrificed, synovial lavage is collected, and keratan sulfate (KS) and sulfated glycosaminoglycan (S-GAG) fragments released into the joint are quantitated. Keratan sulfate is measured by an inhibition ELISA using the method of Thonar (Thonar et al., "Quantitation of Keratan Sulfate in Blood As a Marker of Cartilage Catabolism", Arth. Rheum., Vol. 28, pp.1367-1376 (1985)). Sulfated glycosaminoglycans are measured by first digesting the synovial lavage with streptomyces hyaluronidase and then measuring DMB dye binding using the method of Goldberg (Goldberg et al., "An Improved Method For Determining Proteoglycan Synthesized by Chondrocytes in Culture", Connect Tis. Res., Vol. 24, pp. 265-275 (1990)). For an intravenous (i.v.) study, a compound is solubilized in 1 mL of PEG-400, and for a p.o. study, a compound is administered in 5 mL of fortified com starch per kilogram of body weight. The assay can be similarly carried out with other MMPs such as recombinant human MMP-13.

The effect in protecting against cartilage degradation in arthritic disorders can be determined, e.g., in a surgical model of osteoarthritis described in Arthritis and Rheumatism, Vol. 26, pp. 875-886 (1983).

The anti-arthritic effect can also be determined in other arthritis models described in Laboratory Animal Science, Vol. 39, p. 115 (1989), Journal of Rheumatology, Vol. 30, Suppl.1, pp. 5-9 (1991), Journal of Pharmacology and Toxicology "methods, Vol. 30, pp. 19-25 (1993), and Brit. J. Pharmacol., Vol. 121, pp. 540-546 (1997), Toxicol. Pathol., Vol. 27, pp. 134-142 (1999).

The effect on ulcerations, e.g., ocular ulcerations, can be determined in the rabbit by measuring the reduction of corneal ulceration following an alkali bum to the cornea.

The antitumor effect of the compounds of the invention can be determined, e.g., by measuring the growth of human tumors implanted subcutaneously in Balb/c nude treated mice according to methodology well-known in the art in comparison to placebo treated mice. Illustrative tumors are, e.g., estrogen depende nt human breast carcinoma BT20 and MCF7, human bladder carcinoma T24, human colon carcinoma Colo 205, human lung adenocarcinoma A549 and human ovarian carcinoma NIH-OVCAR3.

The effect on tumor angiogenesis can be determined, e.g., in rats implanted with Walker 256 carcinoma in pellets to stimulate angiogenesis from vessels of the limbus, as described by Galardy et al., Cancer Res., Vol. 54, p. 4715 (1994).

The effect of the compounds of the invention on atherosclerotic conditions can be evaluated using atherosclerotic plaques from cholesterol-fed rabbits which contain activated matrix metalloproteinases, as described by Sukhova et al., Circulation 90, Vol. I, p. 404 (1994). The inhibitory effect on matrix metalloproteinase enzyme activity in rabbit atherosclerotic plaques can be determined by in situ zymography, as described by Galis et al., J. Clin. Invest., Vol. 94, p. 2493 (1994), and is indicative of plaque stabilization. The effect on restenosis and vascular remodeling can be evaluated in the rat ballooned carotid artery model.

The effect on vascular aneurysms, e.g., the inhibition of aneurysm formation, can be determined in experimental models such as APO-E transgenic mice and/or LDL receptor knockout mice.

The effect on demyelinating disorders of the nervous system, such as multiple sclerosis, can be evaluated by measuring the reversal of experimental antioimmune encephalo-myelitis in mice, e.g., as described by Gijbels et al., J. Clin. Invest., Vol. 94, p. 2177 (1994).

As inhibitors of matrix metalloproteinases, primarily MMP-13, the compounds of the invention are particularly useful in mammals as antiinflammatory agents for the treatment of, e.g., osteoarthritis, rheumatoid arthritis, as antitumor agents for the treatment and prevention of tumor growth, tumor metastasis, tumor invasion or progression, and as antiatherosclerotic agents for the treatment and prevention of the rupture of atherosclerotic plaques.

The potential for inducing musculoskeletal side effects can be determined in the rat tendonitis model as follows:

Mini-pumps are implanted subcutaneously into the backs of female Lewis rats (160-180 g) using sterile techniques. The wound is dosed by 9 mm wound clips and sprayed with antiseptic film to prevent infection. Test compounds are dissolved in DMSO:PEG 400 50:50 (v/v) and the solutions loaded into the pumps in a laminar flow hood to maintain sterility. Rats are dosed via constant infusion, with control animals receiving DMSO:PEG 400 vehicle filled mini-pumps. Musculoskeletal changes are assessed by measuring the volume of the hind paws throughout the study and by visually scoring the hind paws on Day 21. The lack of change of hind paw volume is indicative of lack of musculoskeletal side effects.

Typically, compounds of the invention inhibit collagenase-3 (MMP-13) with IC₅₀s in the range of about 0.1-100 nM; and are substantially free of collagenase-1 (MMP-1) inhibition at effective
MMP-13 inhibiting concentrations. The ratio of the IC₅₀ for MMP-1 inhibition to the IC₅₀ for MMP-13 inhibition is typically in the range of about 100-10,000.

The compounds of formula I can be prepared by (a) condensing an amino acid ester of the formula VII 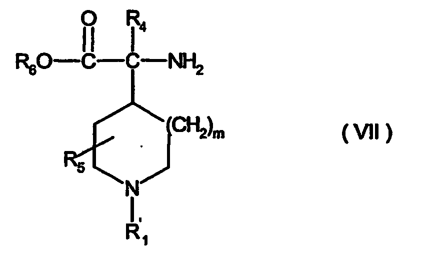 wherein R'₁ is an amino protecting group, e.g., t-BOC, m, R₄ and R₅ have meaning as defined above, and R₆ is hydrogen or a carboxyl protecting group, e.g., lower alkyl or benzyl, with a reactive functional derivative, e.g., the chloride, of the appropriate sulfonic acid of formula VIII 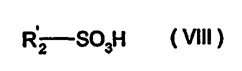 wherein R'₂ is biaryl or aryloxyaryl, e.g., with the corresponding sulfonyl chloride, in the presence of a suitable base, such as triethylamine or N-methylmorpholine using a polar solvent such as methylene chloride, tetrahydrofuran or acetonitrile, and if required, selectively removing the amino protecting group to obtain a compound of formula IX 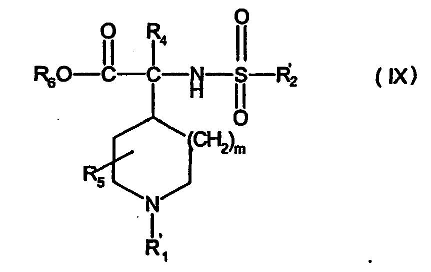
wherein R₄, R₅, R₆, R'₂ and m have meaning as defined above;
and, if required,
   (a) N-substituting the resulting intermediate of formula IX with a reactive derivative corresponding to the R₁ substitutent in formula I; and, if required,
   (b) converting the resulting ester to the corresponding acid by standard methods, e.g., by hydrolysis or debenzylation; and, if required,
   (c) condensing said acid intermediate with hydroxylamine, optionally in protected form, e.g., (O-tetrahydropyranyl, O-trityl or O-t-butyl)-hydroxylamine, to obtain a compound of formula I wherein R is NHOH.
N-substitution on the piperidyl ring of a compound of formula IX can be carried out as follows:
   (a) For conversion of intermediates of formula IX to compounds of formula I wherein R is acyl derived from a carbonic acid (the urethanes of formula III) by
      (i) treating a compound of formula IX, preferably in the branch of N,O-bis-trimethylsilylacetamide, with a compound of the formula X 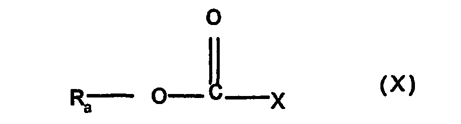 wherein Rₐ has meaning as defined hereinabove and X is a leaving group, such as halo, preferably chloro, in the presence of a base such as triethylamine; or by
      (ii) reacting a compound of formula IX with a reactive derivative of carbonic acid, e.g., phosgene or di(2-pyridyl)carbonate, followed by an alcohol of formula XI

         Rₐ-OH (XI)

         wherein Rₐ has meaning as previously described, in an inert solvent and in the presence of a base such as triethylamine;
   (b) For conversion of intermediates of formula IX to compounds of formula I wherein R is acyl derived form a carboxylic acid (the amides of formula IV), by
      (i) reacting a compound of formula IX, optionally in the presence of N,O-bis-trimethylsilylacetamide, with a reactive functional derivative of a carboxylic acid of the formula XII

         R_{b}-COOH (XII)

         wherein R_{b} has meaning as previously described with, e.g., an acid anhydride or acid chloride, in the presence of a base such as triethylamine, or
      (ii) reacting a compound of formula IX with a carboxylic acid of formula XII in a presence of a condensing agent, e.g., a carbodiimide such as 1-ethyl-3-(3-dimethylaminopropyl)carbodiimide with, e.g., 1-hydroxy-7-azabenzotriazole, and a base such as N-methylmorpholine; or
      (iii) reacting a compound of formula IX with, e.g., chloroacetyl chloride in the presence of a base to yield a chloroacetamide derivative which may be substituted with various nucleophiles, e.g., a primary or secondary amine to yield compounds of formula I wherein R is, e.g., mono- or di-substituted aminoalkanoyl;
   (c) For conversion of intermediates of formula IX to compounds of formula I wherein R is acyl derived from a carbamic acid (the ureas of formula V) by
      (i) treating a compound of formula IX with an isocyanate compound of the formula XIII

         R_{c}-N=C=O (XIII)

         to obtain a compound of formula V wherein R_{c} has meaning other than hydrogen as defined herein above and R_{d} is hydrogen, and if desired further alkylating or acylating the obtained intermediate; or
      (ii) reacting a compound of formula IX with, e.g., phosgene, followed by an amine of the formula XIV 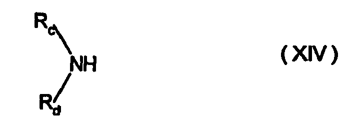 wherein R_{c} and R_{d} have meaning as defined hereinabove in an inert solvent and in the presence of a base, such as triethylamine; or
      (iii) reacting a compound of formula IX with a reactive carbamic acid derivative, e.g., a carbamoyl chloride of the formula XV 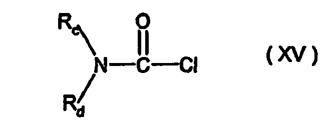
      wherein neither R_{c} nor R_{d} is hydrogen, in the presence of a base;
   (d) For conversion of intermediates of formula IX to compounds of formula I wherein R is substituted sulfonyl, e.g., compounds of formula VI, by treating a compound of formula IX with a reactive functional derivative of a sulfonic acid of the formula XVI

      Rₑ-SO₃H (XVI)

      wherein Rₑ has meaning as previously defined with, e.g., a sulfonic acid halide (e.g., an arylsulfonyl chloride in the presence of a base, such as triethylamine);
   (e) For conversion of intermediates of formula IX to compounds of formula I wherein R₁ is, e.g., lower alkyl or aryl-lower alkyl by
      (i) treating a compound of formula IX with a reactive esterified derivative of the corresponding alcohol, e.g., the bromide, or iodide thereof, in the presence of a base, e.g., potassium carbonate; or
      (ii) by reductive alkylation of a compound of formula IX with a corresponding aldehyde in the presence of a reducing agent, e.g., a borohydride such as sodium triacetoxyborohydride;
      and converting any of the ester intermediates to the corresponding carboxylic acids of formula I using either hydrogenolysis or standard mild methods of ester hydrolysis, preferably under acidic conditions, the method depending on the nature of the esterifying group.

For the above reactions, carboxylic acids of formula IX (wherein R₆ is hydrogen) can be protected *in situ* with bis-trimethylsilylacetamide, using THF as solvent.

The starting materials of formulas VII, VIII and X-XVI are either known in the art and can be prepared by methods known in the art or as described herein.

As to the amino acids of formula VII, the (R)-enantiomers (the D-amino acids) can be prepared via methodology known in the art, as described in the U.S. Patent No. 5,817,822 and references cited therein.

Another method for the preparation of (R)-α-(piperidyl)glycine methyl ester is as follows:

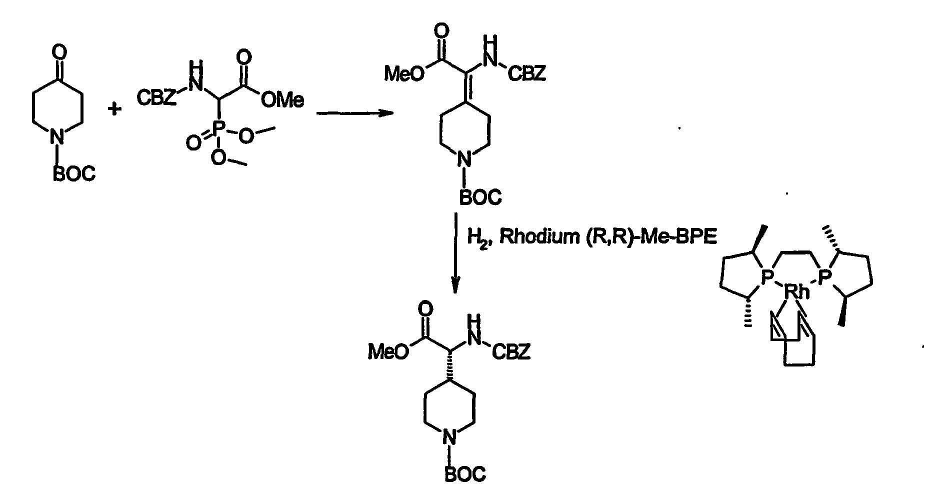

The method involves the asymmetric hydrogenation of the Homer-Emmons condensation product depicted above utilizing a chiral phospholane rhodium complex, e.g., (R,R)-Me-BPE-Rh catalyst (U.S. Patent No. 5,008,457) under hydrogenation conditions. The CBZ protecting group can be removed under standard conditions (namely, hydrogenolysis) and the ester group can be removed under standard conditions, e.g., by treatment with a base such as lithium hydroxide.

The sulfonyl chloride starting materials corresponding to sulfonic acids of formula VIII wherein R'₂ is biaryl can be prepared by methods known in the art, e.g.,
(a) palladium catalyzed Suzuki coupling of an aryl boronic acid with a halo substituted aryl-sulfonic acid in the presence of, e.g., PdCl₂(dppf) catalyst followed by reaction with, e.g., oxalyl chloride; or
(b) treatment of a biaryl compound with n-BuLi/SO₂ followed by reaction with N-chlorosuccinimide, for the preparation of, e.g., a 5-(aryl)-(thiophene or furan)-2-sulfonyl chloride.

The biaryl starting materials can, in turn, be prepared by Suzuki type coupling of an aryl boronic acid with an aryl halide in the presence of PdCl₂ (dppf) catalyst, or by Stille type condensation, e.g., of a tributylstannyl-aryl reagent with an aryl halide in the presence of, e.g., Pd(PPh₃)₄.

The preparation of aryloxyarylsulfonyl chlorides is described in the art, e.g., WO 98/43963.

Compounds of the invention, e.g., of formula I, wherein R₃ is hydrogen, R₁ is acyl and wherein COOH is in protected form (e.g., as benzyl ester), can be converted to compounds wherein R₃ is, e.g., lower alkyl, by treatment with an alkyl halide in the presence of a base, e.g., potassium carbonate, and subsequent removal of the protecting group (hydrogenation for removal of the benzyl group).

Compounds of the invention or intermediates can be converted to related compounds of the invention or intermediates using methodology well known in the art. For example, alkoxy substituted biaryl or aryl derivatives can be dealkylated to the corresponding hydroxy substituted compounds by treatment with, e.g., hydrobromic acid.

For the above mentioned reactions, the preferred solvents, catalysts, reagents and reaction conditions are set forth in the appended illustrative examples.

Depending on the choice of starting materials and methods, the new compounds may be in the form of one of the possible isomers or mixtures thereof, e.g., as substantially pure optical isomers (antipodes), racemates, or mixtures thereof. The aforesaid possible isomers or mixtures thereof are within the purview of this invention.

Any resulting mixtures of isomers can be separated on the basis of the physico-chemical differences of the constituents into the pure geometric or optical isomers, diastereoisomers, racemates, e.g., by chromatography and/or fractional crystallization.

Any resulting racemates of final products or intermediates can be resolved Into the optical antipodes by known methods, e.g., by separation of the diastereoisometric salts thereof, obtained with an optically active acid or base, and liberating the optically active acidic or basic compound. The hydroxamic acids or carboxylic acid intermediates can thus be resolved into their optical antipodes, e.g., by fractional crystallization of d- or 1-(α-methylbenzylamine, cinchonidine, cinchoniine, quinine, quinidine, ephedrine, dehydroabietylamine, brucine or strychnine)-salts; or by enantioselective chromatography.

Finally, acidic compounds of the invention are either obtained in the free form, or as a salt thereof.

Acidic compounds of the invention may be converted into salts with pharmaceutically acceptable bases, e.g., an aqueous alkali metal hydroxide, advantageously in the presence of an ethereal or alcoholic solvent, such as a lower alkanol. From the solutions of the latter, the salts may be precipitated with ethers, e.g., diethyl ether. Resulting salts may be converted into the free compounds by treatment with acids. These or other salts can also be used for purification of the compounds obtained.

In view of the close relationship between the free compounds and the compounds in the form of their salts, whenever a compound is referred to in this context, a corresponding salt is also intended, provided such is possible or appropriate under the circumstances.

The compounds, including their salts, can also be obtained in the form of their hydrates, or include other solvents used for their crystallization.

The pharmaceutical compositions according to the invention are those suitable for enteral, such as oral or rectal, transdermal and parenteral administration to mammals, including man, to inhibit matrix-degrading matalloproteinases, in particular, MMP-13 (collagenase-3), and for the treatment of disorders responsive thereto, comprising an effective amount of a pharmacologically active compound of the invention, alone or in combination, with one or more pharmaceutically acceptable carriers.

The pharmacologically active compounds of the invention are useful in the manufacture of pharmaceutical compositions comprising an effective amount thereof in conjunction or admixture with excipients or carriers suitable for either enteral or parenteral application. Preferred are tablets and gelatin capsules comprising the active ingredient together with a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also; c) binders, e.g., magnesium aluminum silicate, starch paste, gelatin, tragacanth, methylcellulose, sodium carboxymethylcelluose and/or polyvinylpyrrolidone; if desired; d) disintegrants, e.g., starches, agar, alginic acid or its sodium salt, or effervescent mixtures; and/or e) absorbents, colorants, flavors and sweetners. Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1 to 75%, preferably about 1 to 50%, of the active ingredient.

Suitable formulations for transdermal application include an effective amount of a compound of the invention with carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin. Suitable formulations for topical application, e.g., to the skin and eyes, are preferably aqueous solutions, ointments, creams or gels well-known in the art.

The pharmaceutical formulations contain an effective matrix-degrading metalloproteinase inhibiting amount of a compound of the invention as defined above, either alone or in combination with another therapeutic agent, e.g, an antlinflammatory/analgesic agent with cyclooxygenase inhibiting activity, or other antirheumatic agents such as methotrexate, each at an effective therapeutic dose as reported in the art. Such therapeutic agents are well-known in the art.

Examples of antiinflammatory/analgesic agents with cyclooxygenase inhibiting activity are diclofenac, naproxen, ibuprofen, rofecoxib, celecoxib, etoricoxib, valdecoxib, parecoxib, tiracoxib, COX-189 and ABT-963.

In conjunction with another active ingredient, a compound of the invention may be administered either simultaneously, before or after the other active ingredient, either separately by the same or different route of administration or together in the same pharmaceutical formulation.

The dosage of active compound administered is dependent on the species of warm-blooded animal (mammal), the body weight, age and individual condition, and on the form of administration. A unit dosage for oral administration to a mammal of about 50 to 70 kg may contain between about 10 and 1000 mg, advantageously between about 25 and 250 mg of the active ingredient.

The present disclosure also describes methods of using the compounds of the invention and their pharmaceutically acceptable salts, or pharmaceutical compositions thereof, in mammals for inhibiting the matrix-degrading metalloproteinases, in particular collagenase-3, for inhibiting tissue matrix-degradation, and for the treatment of matrix-degrading metalloproteinase dependent conditions as described herein, e.g., inflammation, rheumatoid arthritis, osteoarthritis, also tumors (tumor growth, metastasis, progression or invasion), pulmonary disorders, atherosclerosis and the like described herein. Tumors (carcinomas) include mammalian breast, lung, bladder, colon, prostate and ovarian cancer, and skin cancer, including melanoma and Kaposi's sarcoma.

The following examples are intended to illustrate the invention and are not to be construed as being limitations thereon. Temperatures are given in degrees Centrigrade. If not mentioned otherwise, all evaporations are performed under reduced pressure, preferably between about 15 and 100 mm Hg (=20-133 mbar). The structure of final products, intermediates and starting materials is confirmed by standard analytical methods, e.g., microanalysis and spectroscopic characteristics (e.g., microanalysis and spectroscopic characteristics (e.g., MS, IR, NMR). Abbreviations used are those conventional in the art.
The concentration for [α]_{D} determinations is expressed in mg/ml.

Some of the abbreviations used in the application are:
NMM for N-methylmorpholine
CBZ for benzyloxycarbonyl
BOC for t-butoxycarbonyl
t-MMP-13 for truncated MMP-13
Mca for (7-methoxycoumarin-4-y)acetyl
NFF2 for Mca-Arg-Pro-Lys-Pro-Tyr-Ala-Nva-Trp-Met-Lys(Dnp)-NH₂
Nva for norvaline
NMA for 2-N-methylaminobenzoic acid
Dnp for 2,4-dinitrophenyl
Dppf for (diphenylphospino)ferrocene
DMF for dimethylformamide
THF for tetrahydrofuran
IPA for isopropyl alcohol
TFA for trifluoroacetic acid
OSM for oncostatin M
Rh-IL-1-α for recombinant human interleukin 1-α
HyPro for hydroxyproline
EDCI for 1-(3-dimethylaminopropyl)-3-ethylcarbodi imide hydrochloride
HOAT for 1-hydroxy-7-azabenzotriazole
(R,R)-Me-BPE-Rh for (1,2-Bis(R,R)-2,5-dimethyl-1-phospholidinyl)ethane)(1,5-cyclooctadiene)rhodium(1+trifluoromethanesulfonate

In the tables listing examples, the point of attachment of groups R₁ and R₂ is indicated by a line ending with an asterisk (*).

### Examples

### Typical Procedures for Preparation of Starting Materials

### (a) 2-(4-N,N-Dimethylaminophenyl)-thiophene

To a mixture of 5.44 g (42.5 mmol) of thiophene-2-boronic acid, 4.25 g (21.2 mmol) of 4-bromo-*N*,*N-*dimethylaniline and 22.42 g (212 mmol) of Na₂CO₃ in anhydrous ethanol is added 1.22 g (1.05 mmol) of PdCl₂(dppf). The mixture is heated to 55°C overnight, cooled to room temperature and filtered to remove the sodium carbonate. The solvent is removed *in vacuo* and EtOAc (100 mL) is added to the resulting residue. This mixture is eluted through a short column of silica gel (100 g) using hexanes/EtOAc (4:1). The solvent is removed *in vacuo* to provide a yellow solid which is triturated with hexanes to provide the title compound as a yellow solid.

2-[4-(1-Pyrrolidinyl)-phenyl] thiophene is prepared in a similar fashion starting from thiophene-2-boronic acid and 4-bromo-1-(1-pyrrolidinyl) benzene.

### (b) 5-(4-N,N-Dimethylaminophenyl)-2-thienylsulfonyl chloride

250 mg (1.23 mmol) of 2-(4-*N*,*N*-dimethylaminophenyl)-thiophene is added slowly to neat chlorosulfonic acid (0.81 mL) cooled to 0°C in an ice bath. The reaction mixture is stirred at 0°C for 5 minutes after which the bath is removed and the mixture is stirred for an additional 10 minutes and then it is poured over ice (15 g). The yellow-orange precipitate is collected by vacuum filtration, washed with water and air dried to provide the title compound.

5-[4-(1-Pyrrolidinyl)-phenyl]-2-thienylsulfonyl chloride is prepared in a similar fashion starting from 2-[4-(1-pyrrolidinyl)-phenyl]thiophene.

### (c) 4-(4'-Methoxyphenyl)phenylsulfonyl chloride

A solution of 7.6 g (50 mmol) of 4-methoxyphenylboronic acid, 11.85 g (50 mmol) of 4-bromophenylsulfonic acid, 13.8 g (100 mmol) of potassium carbonate and 3.66 g (5 mmol) of PdCl₂(dppf) in 300 mL of a 1:1 mixture of dimethoxyethane and water under an atmosphere of nitrogen is heated to 80°C for 3.5 hours. The reaction mixture is cooled to room temperature and added to a mixture of 1300 mL of water and 500 mL of diethyl ether. The insoluble precipitate is filtered off. This solid material is then slurried with acetone and filtered to yield a gray solid which is washed with diethyl ether and dried *in vacuo* to give 4-(4'-methoxylphenyl)phenylsulfonic acid. 18.2 mL (208 mmol) of oxalyl chloride is added dropwise over 20 minutes to a suspension of 11 g (41.6 mmol) 4-(4'-methoxyphenyl)phenylsulfonic acid in 1000 mL THF. The mixture is stirred for a further 15 minutes then cooled to 0°C before 16.1 mL (208 mmol) of DMF is added dropwise. The mixture is stirred at room temperature for 18 hours. The reaction is cooled to 0°C and HCl (4 N) added carefully until no more exothermic reaction is observed. Brine and diethyl ether are added and the layers separated. The organic layer is extracted with brine and the combined aqueous layers are extracted with ethyl acetate. The combined organic layers are dried over magnesium sulfate and filtered. The solution is partially purified by filtration through a very short pad of silica. The solvent is removed *in vacuo* and the residue triturated with hexane:diethyl ether 1:1 to give a solid which is filtered off and recrystallized from diethyl ether to give 4-(4'-methoxyphenyl)phenylsulfonyl chloride.

### (d) 5-(3,4-Methylenedioxyphenyl)-thiophene-2-sulfonyl chloride

A solution of 8.3 g (50 mmol) of 3,4-methylenedioxyphenylboronic acid, 2.4 mL (25 mmol) of 2-bromothiophene, and 1 g (1.4 mmol) of PdCl₂(dppf) in 200 mL of dimethoxyethane under an atmosphere of nitrogen is heated to 65°C for 18 hours. The reaction mixture is cooled to room temperature and filtered through Celite. The filtrate is washed with saturated brine (100 mL) and dried over magnesium sulfate. The mixture is filtered and the solvent is evaporated *in vacuo* to give a dark syrup which is purified by column chromatography (silica gel, 5% ethyl acetate/hexane) to give 2-(3,4-methylenedioxyphenyl)thiophene.

To a solution of 2.04 g (10 mmol) 2-(3,4-methylenedioxyphenyl)thiophone in 80 ml of THF cooled to -78°C is added dropwise 6.9 mL of a 1.6 M solution of n-BuLi in hexane. The mixture is stirred under an atmosphere of nitrogen for 45 minutes. Sulfur dioxide is bubbled into the reaction mixture for 25 minutes. The mixture is allowed to warm to 0°C and then to room temperature, and then the solvents are removed *in vacuo.* The residue is suspended in hexane and filtered. The yellow solid is washed with hexane and dried to give lithium 5-(3,4-methylenedioxyphenyl)-thiophene 2-sulfinate.

To a suspension of 0.12 g (0.43 mmol) lithium 5-(3,4-methylenedioxyphenyl)-thiophene-2-sulfinate in methylene chloride cooled to 0°C is added 0.057 g (0.43 mmol) of N-chlorosuccinimide. The mixture is stirred for 15 minutes and then warmed to room temperature and stirred for an additional 15 minutes. The reaction mixture is filtered through a pad of Celite and the solvent is evaporated *in vacuo* to provide the 5-(3,4-methylenedioxyphenyl)-thiophene-2-sulfonyl chloride.

The following sulfonyl chlorides can be prepared in a similar manner:
5-(4-methoxyphenyl)-thiophene-2-sulfonyl chloride;
5(4-ethoxyphenyl)-thiophene-2-sulfonyl chloride;
5-(3,4-dimethoxyphenyl)-thiophene-2-sulfonyl chloride;
5-(4-trifluoromethylphenyl)-thiophene-2-sulfonyl chloride;
5-(4-methylphenyl)-thiophene-2-sulfonyl chloride; and
5-phenyl-thiophene-2-sulfonyl chloride

### (e) 2-(4-isopropoxyphenyl)thiophene-2-sulfonyl chloride

2-(4-Isopropoxyphenyl)-thiophene is prepared using a Mitsunobu reaction (J. Org. Chem., Vol. 55, p. 2244 (1990)). To a solution of 2-(4-hydroxyphenyl)thiophene (2.0 g, 11.3 mmol, 1 eq.) and triphenylphosphine (3.27 g, 12.5 mmol,1.1 eq.) in THF (55 mL) are added 1.0 mL of isopropyl alcohol (0.78 g, 13.1 mmol,1.15 eq.) and 2.0 mL of diethyl azodicarboxylate (DEAD) (2.17 g,12.5 mmol, 1.1 eq.), respectively. The reaction is left to stir overnight for 18 hours. The solution is concentrated and the residue treated with 25 mL of hexane resulting in the formation of a precipitate. The hexane is decanted and the solids are washed further with hexane (4 x 25 mL). The combined hexane washings are filtered through a plug of silica and concentrated to give a 2-(4-isopropoxyphenyl)-thiophene. This is converted to 4-(isopropoxyphenyl)-thiophene-2-sulfonyl chloride as described under (d) above.

The following compounds can be prepared in an analogous fashion:
5-(4-ethoxyphenyl)-thiophene-2-sulfonyl chloride;
5-(4-propoxyphenyl)-thiophene-2-sulfonyl chloride; and
5-(4-methoxyethoxyphenyl)-thiophene-2-sulfonyl chloride.

### (f) 5-(4-Trifluromethylphenyl)-furan-2-sulfonyl chloride

To a mixture of 6.3 mL (20.0 mmol) of 2-tri-n-butylstannylfuran and 2.95 mL (21.0 mmol) of 4-bromobenzotrifluoride in 100 mL of toluene is added 50 mg of Pd(PPh₃)₄. The mixture is heated to reflux under an atmosphere of nitrogen for one hour. A second 50 mg portion of Pd(PPh₃)₄ is added, and the mixture is heated an additional four hours. The reaction is cooled to room temperature and filtered through a pad of Celite and a pad of silica gel. The toluene is evaporated *in vacuo* to give a yellow oil which is purified by column chromatography (silica gel, 5% ethyl acetate/hexane) to give a yellow oil which is, in turn, crystallized from diethyl ether/hexane to give 2-(4-trifluoromethylphenyl)furan.This is converted to 5-(4-trifluoromethylphenyl)-furan-2-sulfonyl chloride as described under (d) above.

Similarly prepared are 5-(3,4-difluorophenyl)-furan-2-sulfonyl chloride and 5-(3,4-methylenedioxyphenyl)-furan-2-sulfonyl chloride.

### (g) 5-(4-Ethoxyphenyl)-thiophene-2-sulfonyl chloride

A solution of 4-ethoxyphenylboronic acid (300 mmol), 5-bromothiophene-2-sulfonic acid (300 mmol), potassium carbonate (600 mmol) and PdCl₂(dppf) (30 mmol) in 1500 ml of a 1:1 mixture of dimethoxyethane and water under an atmosphere of nitrogen is heated to 80°C for 4 hours. The reaction mixture is cooled to room temperature and added to a mixture of 3000 mL of water and 1500 mL of diethyl ether. The insoluble precipitate is filtered off. This solid material is then slurried with 1000 mL of a 1:1 mixture of acetone and diethyl ether and filtered to yield a solid which is washed with diethyl ether and dried *in vacuo* to give 5-(4-ethoxyphenyl)thiophene-2-sulfonic acid.

Oxalyl chloride (1456 mmol) is added dropwise over 20 minutes to a suspension of 5-(4-ethoxyphenyl)thiophenesulfonic acid (290 mmol) in 1600 mL THF. The mixture is stirred for a further 15 minutes, then cooled to 0°C before DMF (1456 mmol) is added dropwise. The mixture is stirred at room temperature for 18 hours. The reaction is cooled to 0°C and HCl (4 N) added carefully until no more exothermic reaction is observed. Brine and diethyl ether are added and the layers separated. The organic layer is extracted with brine and the combined aqueous layers are extracted with ethyl acetate. The combined organic layers are dried over magnesium sulfate and filtered. The solution is partially purified by filtration through a very short pad of silica. The solvent is removed *in vacuo* and the residue triturated with hexane/diethyl ether (1:1) to give a solid which is filtered off and recrystallized from diethyl ether to give 5-(4-ethoxyphenyl)thiophene-2-sulfonyl chloride.

### (h) 5-(4-Triffuromethoxyphenyl)-thiophene-2-sulfonyl chloride

A mixture of 4-(trifluromethoxy)bromobenzene (4.54 g,18.8 mmole), thiophene-2-boronic acid (4.82 g, 37.6 mmol) and Na₂CO₃ (12 g, 112.8 mmol) in EtOH (150 mL) is stirred at room temperature. Then, [1,1'-*bis* (diphenylphosphnio)-ferrocene]dichloropalladium(ii) complex with dichloromethane (1:1) (1.1 g, 0.94 mmol) is added and the reaction mixture is heated at 65°C for 18 hours. The reaction mixture is cooled to room temperature, filtered through celite and concentrated *in vacuo.* The residue is dissolved in EtOAc and silica gel is added. The EtOAc is evaporated in vacuo and the gel is placed on a column of silica gel and eluted with hexane, then EtOAc/Hexane (1:9) to give 2-(4-trifluromethoxyphenyl)-thiophene as a solid.

A solution of 2-(4-trifluoromethoxyphenyl)-thiophene (2.15 g, 8.8 mmol) in THF (110 mL) is stirred at-78°C and n-BuLi (6.06 mL,1.6 M in hexane) is added. The mixture is stirred at -78°C for 45 minutes, then SO₂ gas is bubbled through the solution for 15 minutes, stirred at -78°C for 1 hour, warmed to room temperature and stirred for another 15 minutes. Hexane is added to the mixture and the product is filtered. The precipitate is washed with hexane to give lithium 5-(4-trifluromethoxyphenyl)-thiophene-2-sulfinate.

To a suspension of lithium 5-(4-trifluromethoxyphenyl)-thiophene-2-sulfinate (960 mg, 3.06 mmol) in CH₂Cl₂ (30 mL) is added N-chlorosuccinimide (408 mg, 3.06 mmol) and the mixture is stirred at 0°C for 15 minutes, then warmed to room temperature and stirred for an additional 15 minutes. The reaction mixture is filtered, rinsed with CH₂Cl₂, and concentrated *in vacuo.* Flash chromatography using EtOAc/Hexane (1:1) yields 5-(4-trifluromethoxyphenyl)-thiophene-2-sulfonyl chloride.

### (i) (R)-α-N-BOC-4-piperidinyl)-glycine

### Step 1

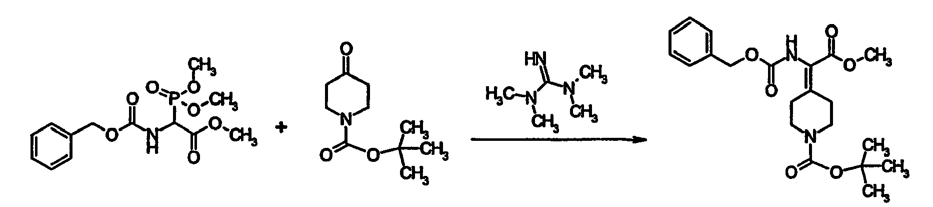

To a solution of 9.94 g (0.030 mmol) of N-CBZ-α-phosphonoglycine trimethyl ester in 20 mL of tetrahydrofuran under nitrogen is added tetramethylguanidine (4.5 g, 0.039 mmol) and the solution is stirred for 15 minutes. A solution of N-BOC-4-piperidone (16.74 g, 0.084 mmol) in tetrahydrofuran is added via addition funnel over 5 minutes and the solution is stirred at room temperature for 21 hours. Tetrahydrofuran is removed via rotary evaporator and ethyl acetate (100 mL) is added. The organic solution is washed with 5% aqueous citric acid solution (150 mL), saturated sodium bicarbonate solution (50 mL), saturated sodium chloride solution (50 mL), dried over magnesium sulfate, and evaporated to give an oil, which is dissolved in ethyl acetate (12 mL). Hexane (50 mL) is added to precipitate the crude product which is filtered off and recrystallized from ethyl acetate/hexane (1:4 ratio) to yield 4-[CBZ-amino)-(methoxycarbonyl)-methylene]-N-BOc-piperidine-1-carboxylic acid t-butyl ester as a white solid; m.p. 101.5-102.6°C.

### Step 2

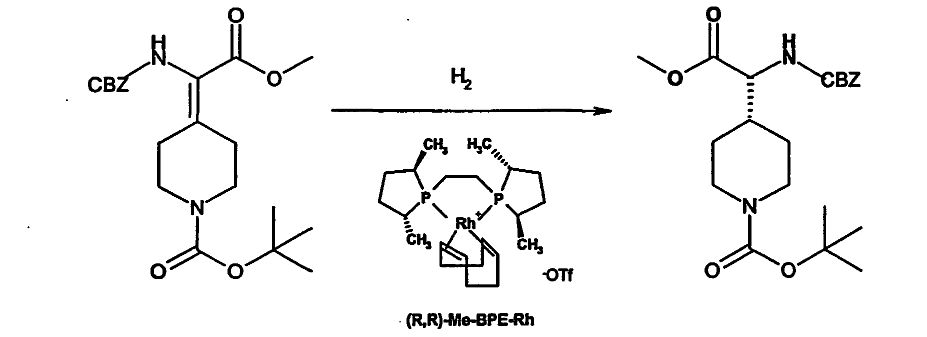

A Parr bottle is charged with product from Step 1 (0.37 g, 0.9 mmol) and degassed MeOH (40 mL) under nitrogen purge. To this colorless solution, (R,R)-Me-BPE-Rh catalyst (10 mg) is quickly added. The resulting solution is evacuated, and then refilled with nitrogen for three cycles. The solution is stirred under 90 psi of hydrogen gas at room temperature over 72 hours. The mixture is concentrated on a rotary evaporator to remove MeOH. The residue is redissolved into ethyl acetate (20 mL) and filtered through a pad of silica gel (3 g) to remove the catalyst, and the filter cake is rinsed with ethyl acetate (20 mL). The combined filtrate is concentrated to afford N-CBZ-(R)-α-(N-BOC-4-piperidinyl)-glycine methyl ester as an oil: Rf = 0.36 (Hexane/EtOAc 1:1); [a]²⁵_{D}-20.7 (c =1.05, CHCl₃); chiral HPLC 94% e.e.: (+)-enantiomer, 3%, Rt 7.21 minutes, (-)-enantiomer, 97%, Rt 10.04 minutes (Chiralcel OD column, Hexane/PA/TFA 9/1/0.1%, flow rate 1.5 mL/min).

### Step 3

A solution of the product from Step 2 (2.8 g, 6.9 mmol) and MeOH (103mL) is cooled to 5°C with an ice bath. A solution of 1 N LiOH (35 mL, 35 mmol, prepared from 1.5 g of LiOH·H₂O in 33.5 mL of H₂O) is added and the mixture is allowed to warm up to room temperature and stirred for another 20 hours. The reaction mixture is neutralized with 1 N KHSO₄ solution, concentrated *in vacuo* to remove MeOH, and redissolved into ethyl acetate. The pH of the aqueous layer is adjusted to 2 with 2 N KHSO₄ and the organic layer is separated. The aqueous layer is further extracted with ethyl acetate (2 x 50 mL). The combined ethyl acetate layers are washed with 50 mL of brine, dried over MgSO₄, filtered through celite, and concentrated under vacuum to give (N-BOC-4-piperidinyl)-glycine as a white foamy solid: [α]²⁵_{D}-18.6 (c = 1.07, CHCl₃) chiral HPLC 90% e.e.: (+)-enantiomer, 5%, Rt 5.72 minutes, (-)-enantiomer, 95%, Rt 8.54 minutes (Chiralcel OD Hexane/IPA/TFA 9/1.0.1 %, flow rate 1.5 mUmin).

### Step 4

A Parr bottle is charged with 5% Pd/C (0.27 g) under nitrogen atmosphere. A solution of the product of Step 3 (1.25 g, 3.2 mmol) in MeOH (14 mL) and H₂O (8 mL) is added under nitrogen purge. The mixture is evacuated and then refilled with nitrogen three times, then evacuated and refilled with hydrogen for another three times. The mixture is hydrogenated under 52 psi hydrogen gas at room temperature for 3 hours. The mixture is filtered and the catalyst cake is rinsed with EtOH (100 mL). The filtrate is concentrated under vacuum to azeotropically remove H₂O. The gray solid residue is suspended in MeOH (20 mL), stirred at 60°C for 2 hours, cooled to 0°C; and stirred for an additional 1 hour. The mixture is filtered and the solid cake is rinsed with cold MeOH (10 mL). The solid is dried under vacuum to obtain (R)-α-(N-BOC-4-piperidinyl)-glycine.

### Example 1

To (R)-α-(*N*-BOC-4-piperidinyl)-glycine (2.77 mmol) and 5-(trifluromethylphenyl)-thiophene-2-sulfonyl chloride (2.77 mmol) in CH₂Cl₂ (30 mL) is added Et₃N (6.93 mmol). The reaction mixture is allowed to stir at room temperature overnight. The reaction mixture is allowed to stir at room temperature overnight. The reaction mixture is quenched with 10% citric acid and the aqueous phase is extracted with CH₂Cl₂ (x2). The combined organic phases are dried over MgSO₄, filtered, and concentrated *in vacuo.* The residue is purified by flash chromatography using a gradient solvent system of 2% to 5% to 10% MeOH/CH₂Cl₂ to give a solution of (α*R*)-1-BOC-α-[[[5-(4-trifluoromethyl-phenyl)-2-thienyl]sulfonyl]amino]-4-piperidineacetic acid ; MS (M-1) : 547.1.

### Reference-Example 2

A solution of (α*R*)-1-BOC-α-[[[5-(4-trifluoromethyl-phenyl)-2-thienyl]sulfonyl]amino]-4-piperidineacetic acid (1.49 mmol) in CH₂Cl₂ is cooled to 0°C. Then HCl(g) is bubbled in for 15 minutes and the reaction is allowed to stir for an additional hour. The solvent is removed *in vacuo* to give (α*R*)-α-[[[5-(4-trifluoromethylphenyl)-2-thienyl]sulfonyl]amino]-4-piperidineacetic acid hydrochloride.

Similarly prepared are, e.g., the following:

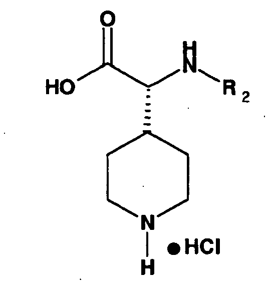

| **Reference-Example** | **R₂** |
|---|---|
| **(a)** | 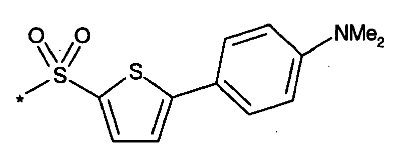 |
| **(b)** | 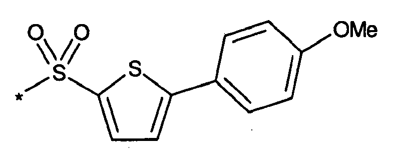 |
| **(c)** | 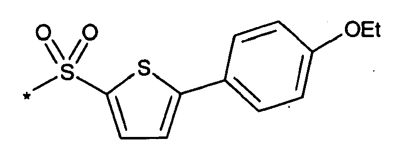 |
| **(d)** | 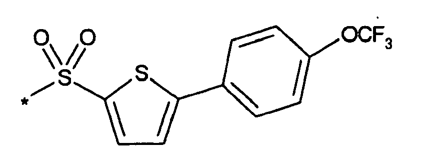 |

### Reference-Example 3

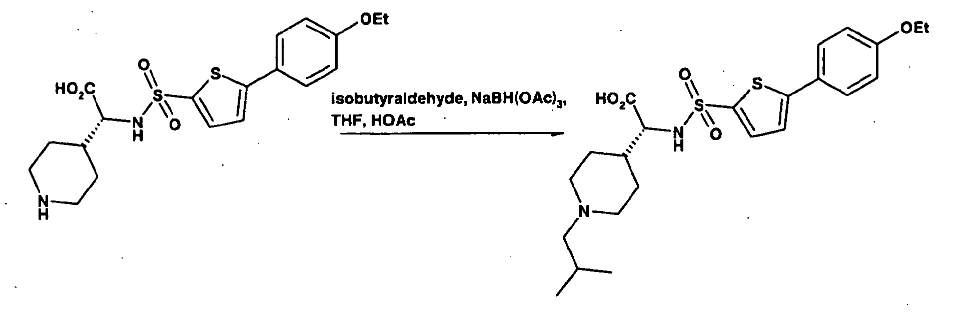

To a solution of 0.067 g (0.15 mmol) of (α*R*)-α-[[[5-(4-ethoxyphenyl)-2-thienyl]sulfonyl]amino]-4-piperidineacetic acid hydrochloride in 10 mL of THF is added 0.015 mL (0.16 mmol) of isobutyraldehyde. This is followed by the addition of 0.046 g (0.22 mmol) of sodium triacetoxyborohydride. The reaction is stirred for 14 hours at room temperature. The mixture is diluted with saturated aqueous sodium bicarbonate, and extracted three times with 10 mL of CH₂Cl₂. The combined organic layers are washed with 10 mL of saturated aqueous brine and then dried over sodium sulfate. The solvents are removed *in vacuo* to give an oil which is purified by column chromatography (LiChroprep^{®} DIOL, CH₂Cl₂) to give (α*R*)-1-(2-methylpropyl)-a-[[[5-(4-ethoxyphenyl)-2-thienyl]sulfonyl]amino]-4-piperidineacetic acid (m.p. 220-225°C (d)); MS (M-1) : 479.2.

Similarly prepared are the following:

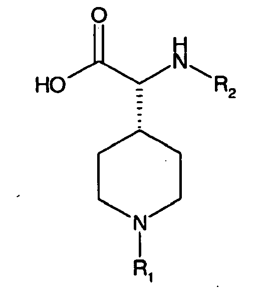

| **Reference-Example** | **R₁** | **R₂** | **MS Found** |
|---|---|---|---|
| **(a)** | 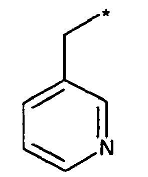 | 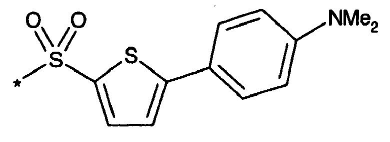 | |
| **(b)** | 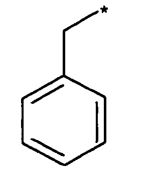 | 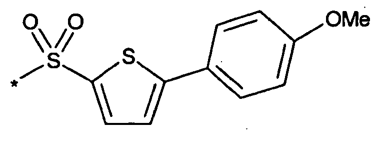 | M+1: 501.2 |
| **(c)** | 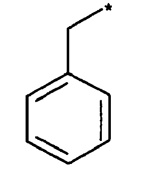 | 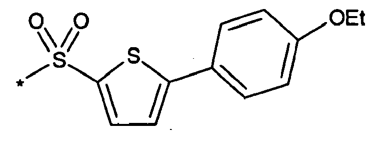 | M-1 : 513.2 |
| **(d)** | 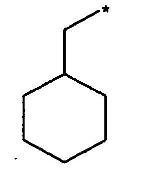 | 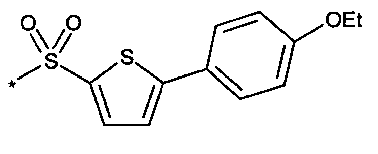 | M+1: 521 |
| **(e)** | 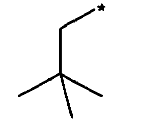 | 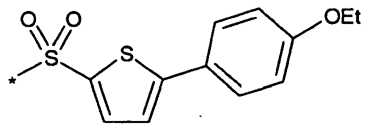 | M-1: 493 |
| **(f)** | 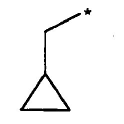 | 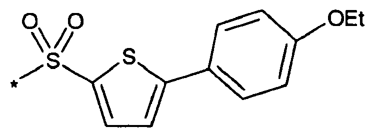 | M+1: 479 |
| **(g)** | 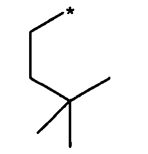 | 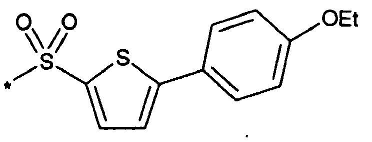 | M+1: 509 |

| **Example** | **R₁** | **R₂** | **MS Found** |
|---|---|---|---|
| **(h)** | 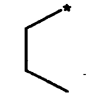 | 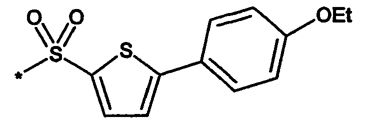 | M+1: 467 |
| **(i)** | 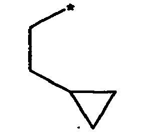 | 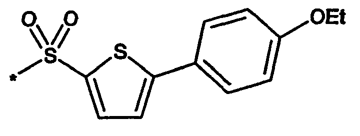 | M+1: 493 |

### Example 4

(a) Typical procedure for the direct acylation of piperidine compounds of formula I wherein R₁ is hydrogen with acid chlorides, chloroformates, isocyanates, carbamoyl chlorides and sulfonyl chlorides. This procedure can be automated using the AutoChem method (Tommasi, et. al., J. Combi., Chem., Vol. 2, No. 5, pp. 447-449 (2000)).

To a solution of a piperidine compound of formula I wherein R₁ is hydrogen (0.05 mmol in 0.5 mL DMF) is added NMM (0.1 mmol, neat), and then the acylating agent (0.05 mmol, neat). The reaction mixture is allowed to stir at room temperature for 12 hours. TFA (50 µL) is added to quench and acidify the mixture and the the product is isolated by reversed-phase HPLC chromatography (YMC C-8 column, 5 mm, 20 x 50 mm, eluted with a linear gradient of 10-90% MeCN/water over 15 minutes). The solvents are removed *in vacuo* to provide the product.

(b) Typical procedure for the acylation of piperidine compounds of formula I wherein R₁ is hydrogen with acid chlorides, chloroformates, isocyanates, carbamoyl chlorides and sulfonyl chlorides with *in situ* protection using *N,O-bis*-trimethylsilyl acetamide.

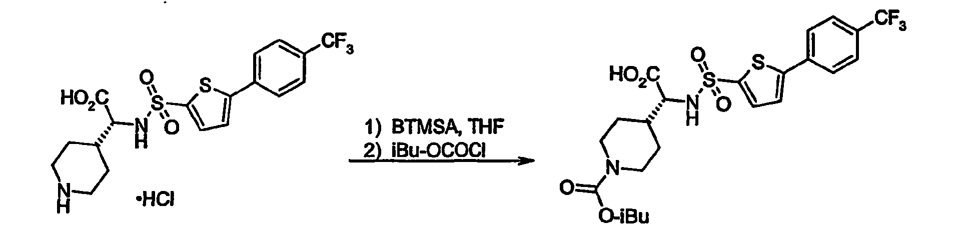

To a solution of 0.24 g (0.5 mmol) of (α*R*)-α-[[[5-(4-trifluoromethylphenyl)-2-thienyl]-sulfonyl]amino]-4-piperidineacetic acid hydrochloride in 10 mL of THF is added 0.49 mL (2.0 mmol) of *N,O-bis*-trimethylsilylacetamide (BTMSA). The reaction is stirred under a nitrogen atmosphere for 0.5 hours at room temperature. Then 0.13 mL (1.0 mmol) of isobutyl chloroformate is added and the reaction is stirred for an additional 14 hours. The solvents are removed *in vacuo* and to the residue is added 10 mL of water. The mixture is made basic by adding saturated aqueous sodium bicarbonate and then extracted with 10 mL of diethyl ether. The aqueous layer is acidified to pH 3 with a solution of 10% aqueous citric acid and extracted three times with 10 mL of ethyl acetate. The combined organic layers are dried over sodiu m sulfate. The solvents are removed *in vacuo* to give an oil which is purified by trituration with Et₂O/EtOAc (10:1) or by column chromatography (LiChroprep^{®} DIOL, 50% CH₂Cl₂/hexane followed by 100% CH₂Cl₂) to give (α*R*)-1-[(2-methylpropoxy)carbonyl]-α-[[[5-(4-trifluoromethylphenyl)-2-thienyl]sulfonyl]amino]-4-piperidineacetic as a yellow solid; m.p. 190-192°C; MS (M-1): 547.1.

(c) Typical procedure for chloroacetylation of a compound of formula I wherein R₁ is hydrogen followed by nucleophilic displacement.

To a solution of (α*R*)-α-[[[5-(4-methoxyphenyl)-2-thienyl]sulfonyl]amino]-4-piperidineacetic acid (500 mg, 1.12 mmol) in THF (10 mL) at room temperature is added bis-trimethylsilylacetamide (909 mg, 4.48 mmol). The mixture is stirred for 4 hours, then chloroacetyl chloride is added (253 mg, 2.24 mmol) and the reaction is allowed to stir overnight. The solvent is removed *in vacuo* and the residue is dissolved in EtOAc and extracted with 1 N HCl. The organic layer is washed with saturated NaCl and then dried with Na₂SO₄. Removal of the solvent *in vacuo* provides (α*R*)-1-(chloroacetyl)-α-[[[5-(4-methoxyphenyl)-2-thienyl]sulfonyl]amino]-4-piperidineacetic acid as a white foam.

To a solution of (α*R*)-1-(chloroacetyl)-α-[[[5-(4-methoxyphenyl)-2-thienyl]sulfonyl]-amino]-4-piperidineacetic acid (140 mg, 0.29 mmol) in acetonitrile (5 mL) at room temperature is added 4-methylpiperazine (86 mg, 0.86 mmol). The mixture is stirred overnight and then concentrated *in vacuo.* Ethanol (5 mL) is added to the residue and the mixture is stirred for 2 hours. The white precipitate, is isolated by vacuum filtration, washed with Ethanol and dried *in vacuo* to provide (α*R*)-1-(4-methylpiperazinoacetyl)-α-[[[5-(4-methoxyphenyl)-2-thienyl]-sulfonyl]amino]-4-piperidineacetic acid; MS (M+1) : 551.3.

### Example 5

Prepared by procedures similar to those described in previous examples are the following amides, using the appropriate acid chloride or anhydride or using chloroacetyl chloride followed by nucleophilic displacement.

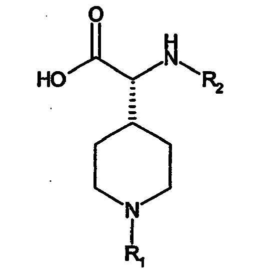

| **Example** | **R₁** | **R₂** | **MS Found** |
|---|---|---|---|
| **(a)** | 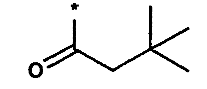 | 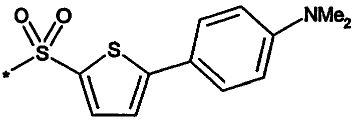 | M+1 : 522.1 |
| **(b)** | 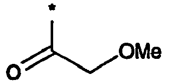 | 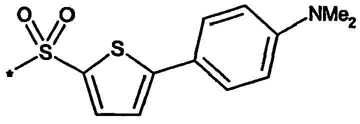 | M+1 : 496.3 |
| **(c)** | 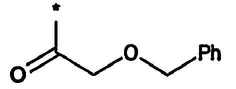 | 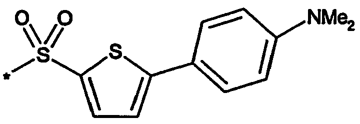 | M+1 : 572.4 |
| **(d)** | 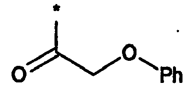 | 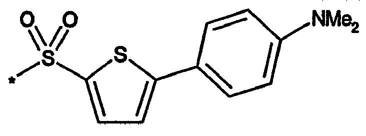 | M+1 : 558.3 |
| **(e)** | 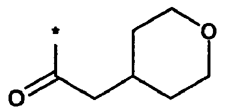 | 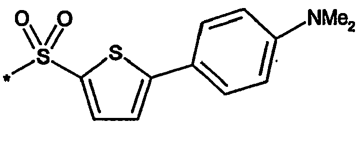 | M+1 : 550.3 |
| **(f)** | 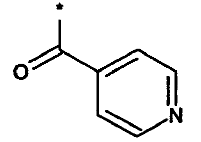 | 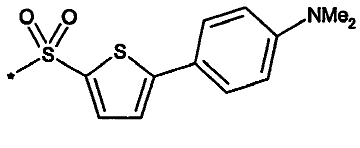 | M-1 : 527.3 |
| **(g)** | 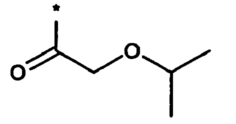 | 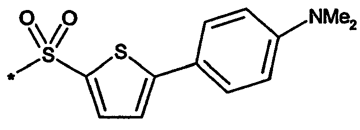 | M+1 : 525 M-1 : 523 |
| **(h)** | 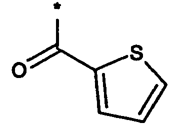 | 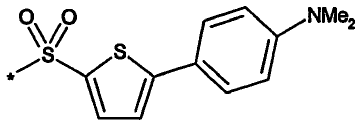 | M+1 : 534.1 |
| **(i)** | 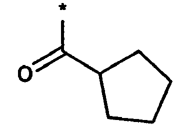 | 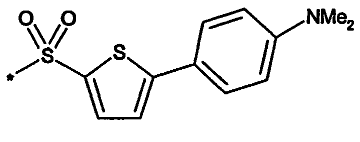 | M+1 : 520.2 |
| **(j)** | 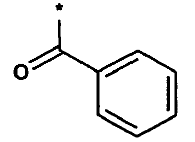 | 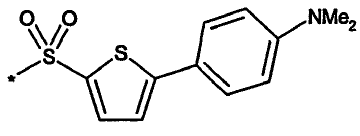 | M+1 : 528.2 |
| **(k)** | 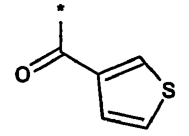 | 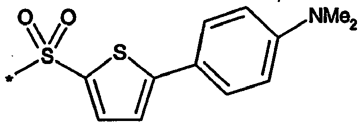 | M+1 : 534.1 |
| **(l)** | 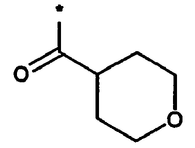 | 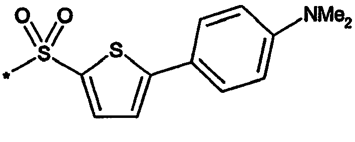 | M+1 : 536.2 |
| **(m)** | 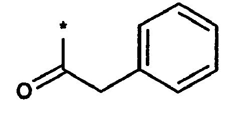 | 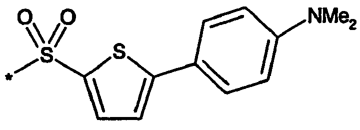 | M+1 : 542.3 |
| **(n)** | 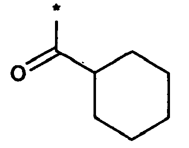 | 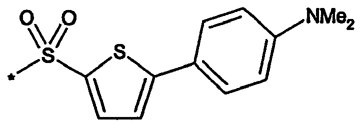 | M+1 : 534.5 |
| **(o)** | 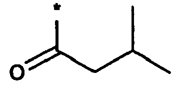 | 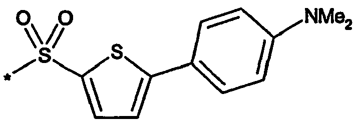 | M+1 : 508.3 |
| **(p)** | 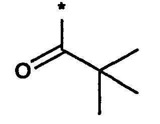 | 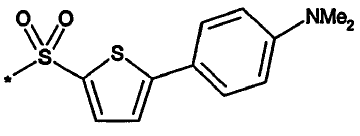 | M+1 : 508.3 |
| **(q)** | 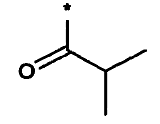 | 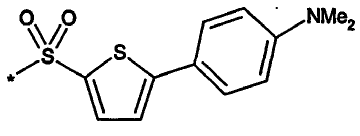 | M+1 : 494.3 |
| **(r)** | 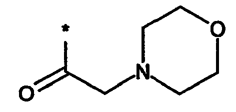 | 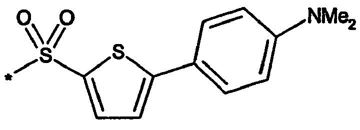 | M+1 : 551.2 |
| **(s)** | 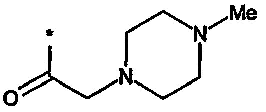 | 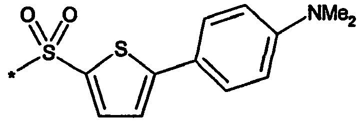 | M+1 : 564.2 |
| **(t)** | 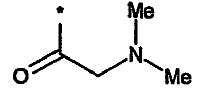 | 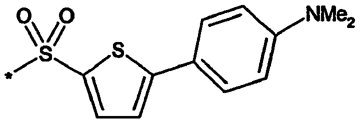 | M+1 : 509.2 |
| **(u)** | 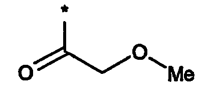 | 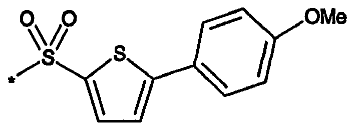 | M+1 : 483.3 |
| **(v)** | 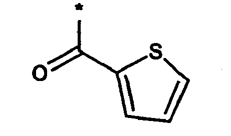 | 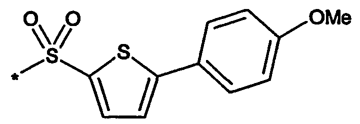 | M+1 : 521.2 |
| **(w)** | 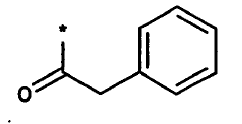 | 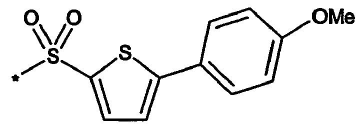 | M+1 : 529.3 |
| **(x)** | 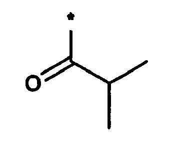 | 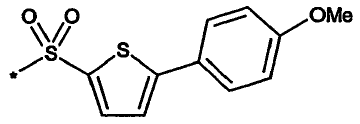 | M+1 : 481.3 |
| **(y)** | 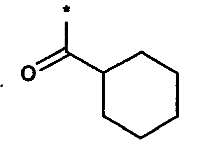 | 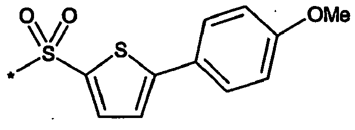 | M+1 : 521.4 |
| **(z)** | 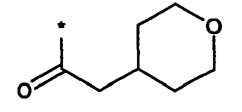 | 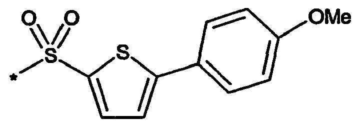 | M+1 : 537.3 |
| **(aa)** | 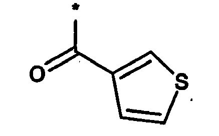 | 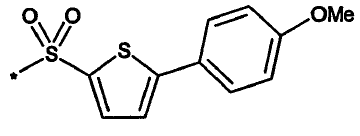 | M+1 : 5621.3 |
| **(ab)** | 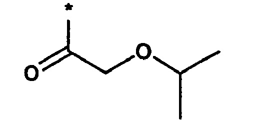 | 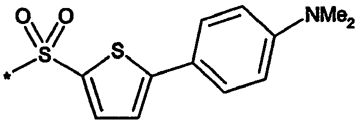 | M+1 : 524.19 |
| **(ac)** | 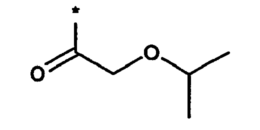 | 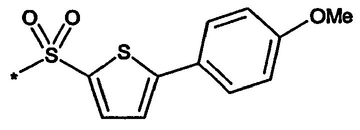 | M+1 : 511.2 |
| **(ad)** | 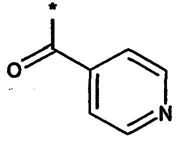 | 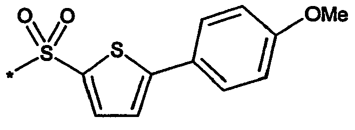 | M+1 : 516.2 |
| **(ae)** | 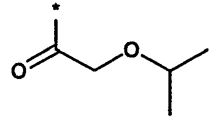 | 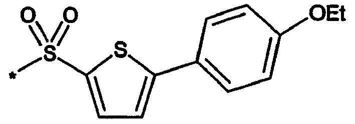 | M+1 : 525.3 |
| **(af)** | 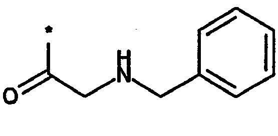 | 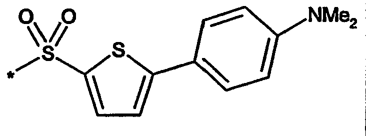 | M+1 : 571.2 |
| **(ag)** | 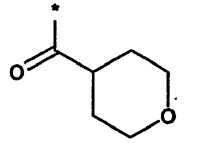 | 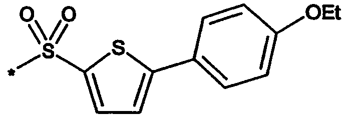 | M+1 : 537.2 |
| **(ah)** | 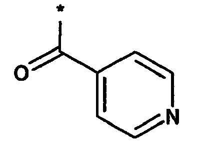 | 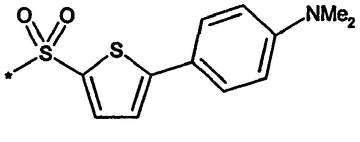 | M+1 : 529.1 |
| **(ai)** | 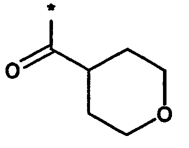 | 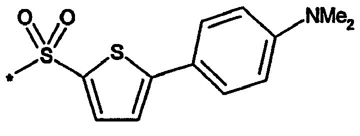 | M+1 : 536.2 |
| **(aj)** | 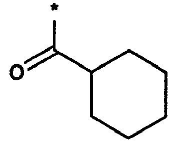 | 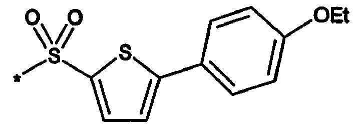 | M+1 : 535.4 |
| **(ak)** | 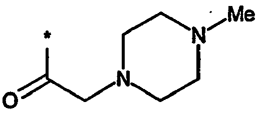 | 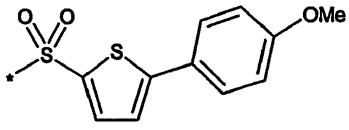 | M+1 : 551.3 |
| **(al)** | 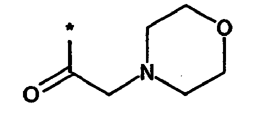 | 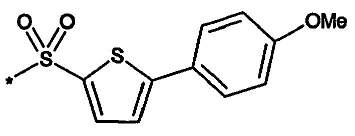 | M+1 : 538.2 |
| **(am)** | 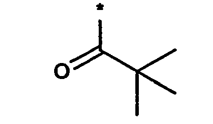 | 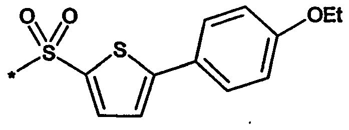 | M+1 : 509.4 |
| **(an)** | 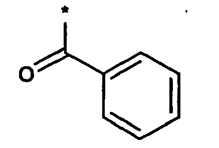 | 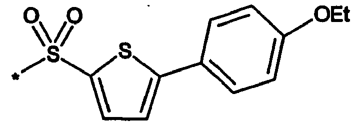 | M+1, M+NH4 529.1, 546.1 |
| **(ao)** | 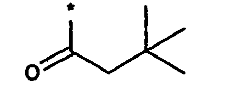 | 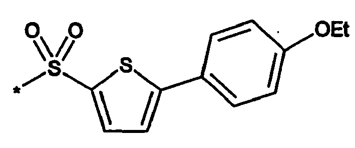 | M+1, M+NH4 523.1, 540.1 |
| **(ap)** | 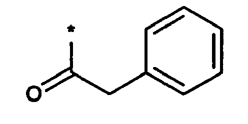 | 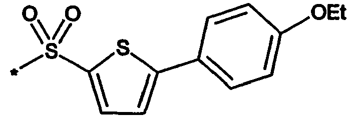 | M+1 : 543.1 |
| **(aq)** | 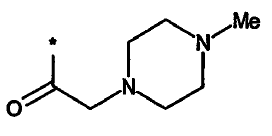 | 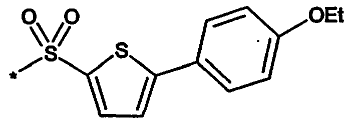 | M+1 : 565.4 |

### Example 6

Prepared by procedures similar to those previously described are the following carbamates, using the appropriate substituted chloroformate:

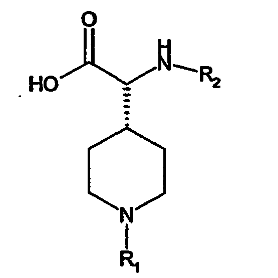

| **Example** | **R₁** | **R₂** | **MS Found** |
|---|---|---|---|
| **(a)** | 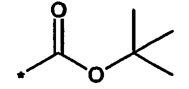 | 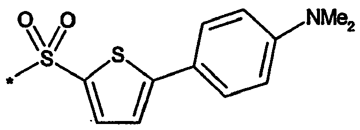 | (M-H): 522.6 |
| **(b)** | 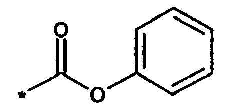 | 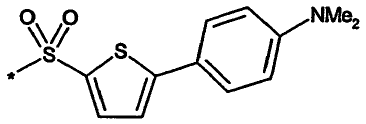 | |
| **(c)** | 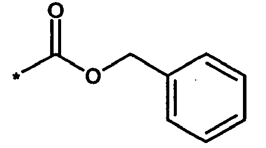 | 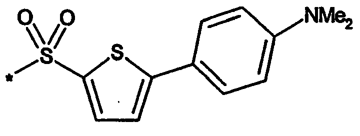 | |
| **(d)** | 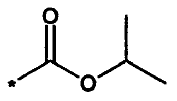 | 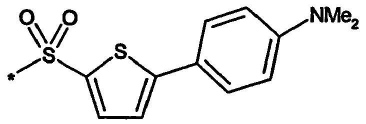 | |
| **(e)** | 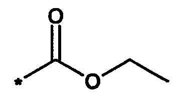 | 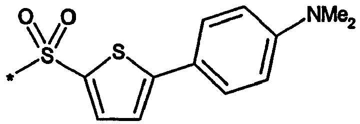 | |
| **(f)** | 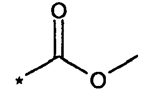 | 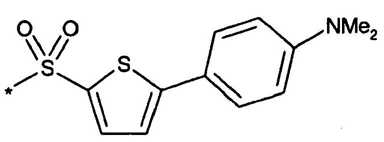 | |
| **(g)** | 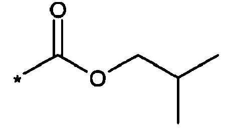 | 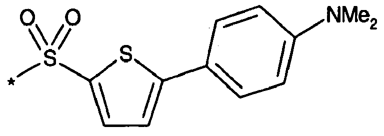 | M+1 : 524.2 |
| **(h)** | 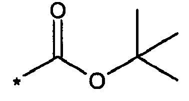 | 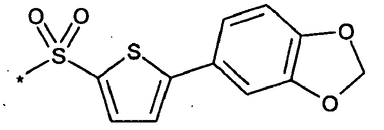 | M-1 : 523.2 |
| **(i)** | 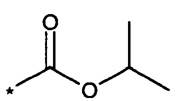 | 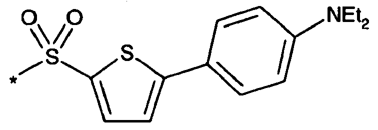 | M+1 : 552.1 |
| **(j)** | 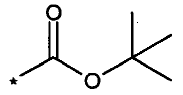 | 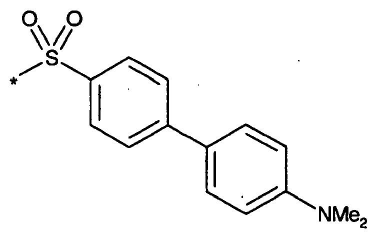 | M+1 : 518.2 |
| **(k)** | 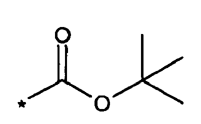 | 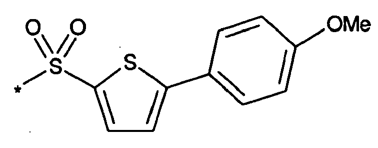 | M+1 : 511.1 |
| **(l) Reference-Example** | 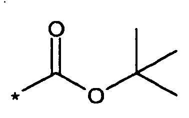 | 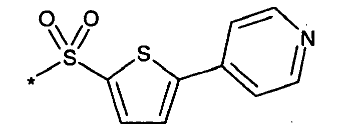 | M+1 : 482.14 |
| **(m)** | 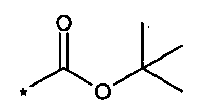 | 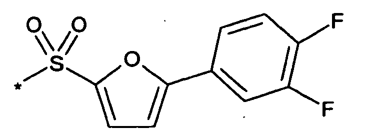 | M-1 : 499.2 |
| **(n)** | 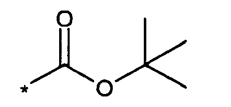 | 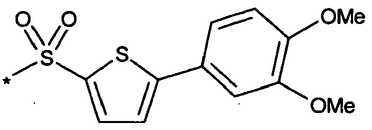 | M-1 : 539.2 |
| **(o) Reference-Example** | 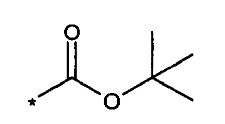 | 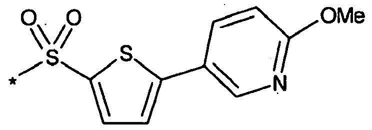 | M-1 : 510.3 |
| **(p)** | 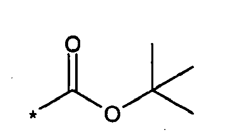 | 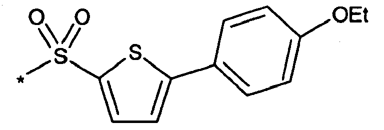 | M+1 : 525.4 |
| **(q) Reference-Example** | 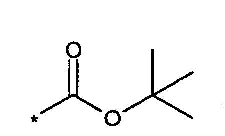 | 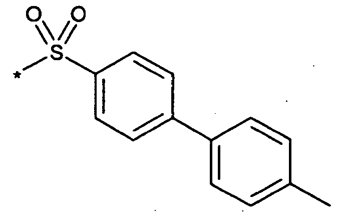 | M+1 : 489.2 |
| **(r) Reference-Example** | 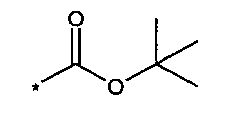 | 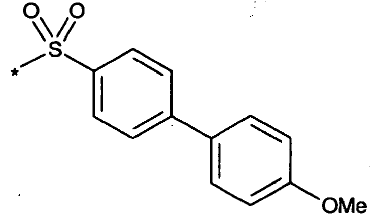 | M+1 : 505.2 |
| **(s)** | 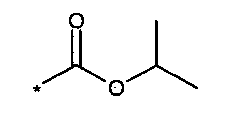 | 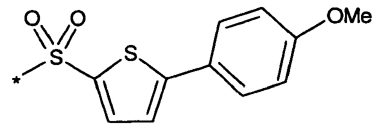 | M+1, M+NH4: 497.1, 514.2 |
| **(t)** | 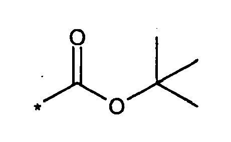 | 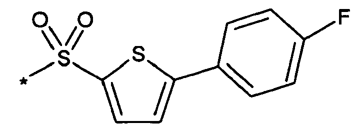 | M+1: 499.1 |
| **(u)** | 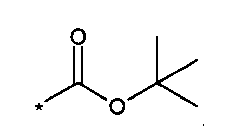 | 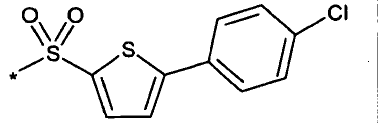 | M+1 : 515.1 |
| **(v)** | 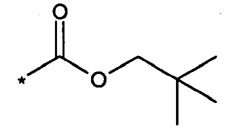 | 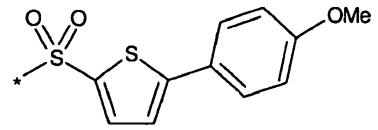 | M+1, M+NH4: 525.1, 542.2 |
| **(w)** | 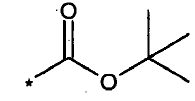 | 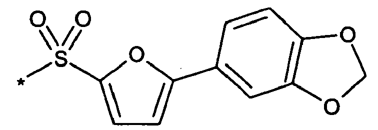 | M-1 : 507.2 |
| **(x)** | 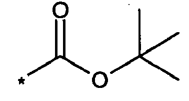 | 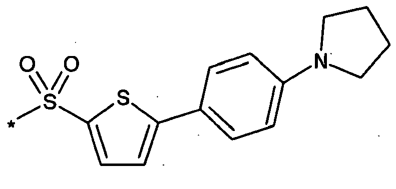 | M+1 : 550.2 |
| **(y)** | 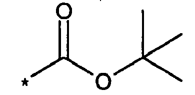 | 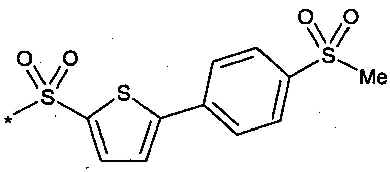 | M+1 : 559.3 |
| **(z)** | 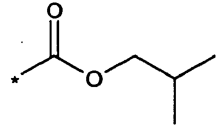 | 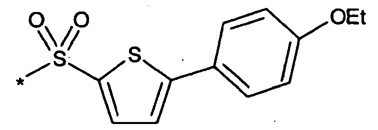 | M-1 : 525.3 |
| **(aa)** | 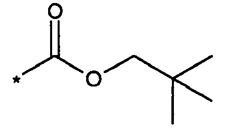 | 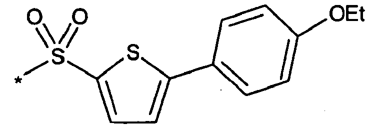 | M+1, M+NH4: 539.4, 556.4 |
| **(ab) Reference-Example** | 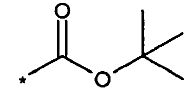 | 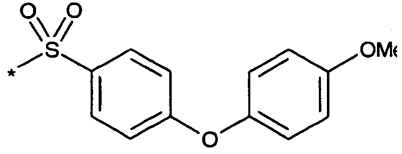 | M+1 : 521.4 |
| **(ac) Reference-Example** | 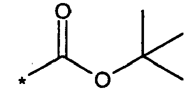 | 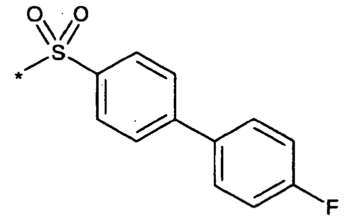 | M+1 : 493.4 |
| **(ad)** | 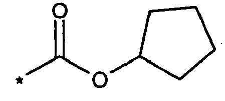 | 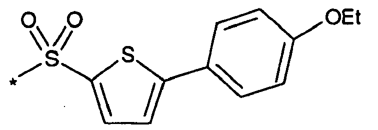 | M-1 : 537.2 |
| **(ae)** | 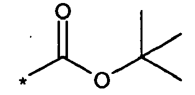 | 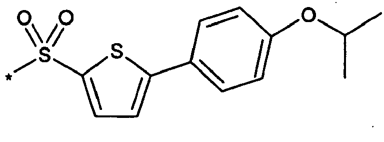 | M-1 : 537.3 |
| **(af)** | 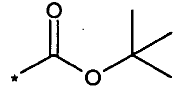 | 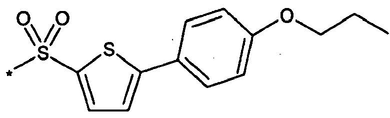 | M-1 : 537.3 |
| **(ag)** | 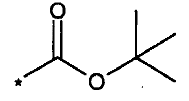 | 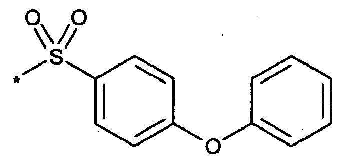 | M+1 : 491.1 |
| **(ah)** | 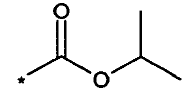 | 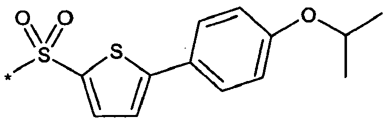 | M-1 : 523.2 |
| **(ai)** | 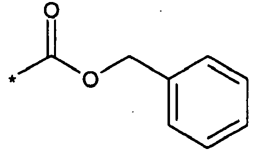 | 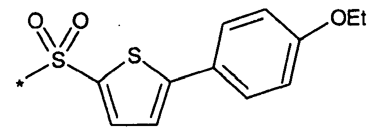 | M+NH4: 576.1 |
| **(aj) Reference-Example** | 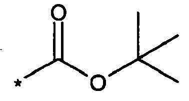 | 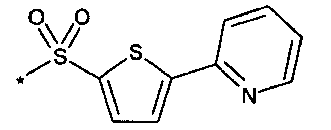 | M+1: 482.4 |
| **(ak)** | 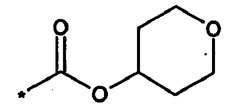 | 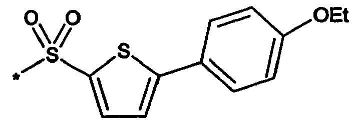 | M+NH4 : 570.1 |
| **(al)** | 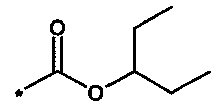 | 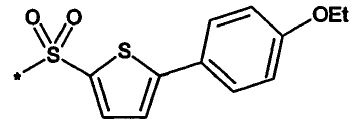 | M+NH4: 556.3 |
| **(am)** | 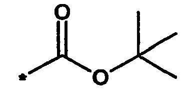 | 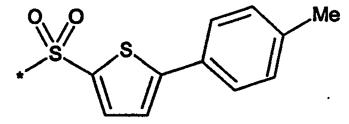 | M-1 : 493.1 |
| **(an)** | 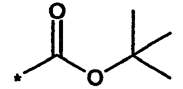 | 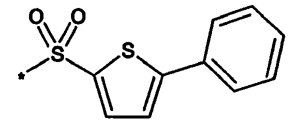 | M-1 : 479.1 |
| **(ao)** | 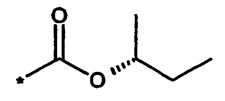 | 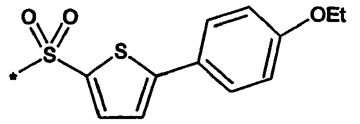 | M+NH4: 542.1 |
| **(ap)** | 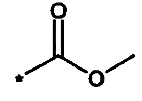 | 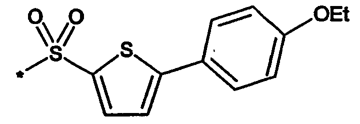 | M+1 : 483 |
| **(aq)** | 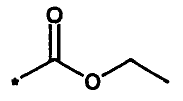 | 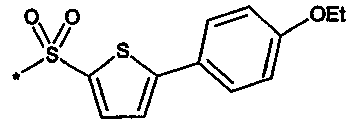 | M+1 : 497 |
| **(ar)** | 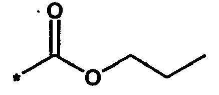 | 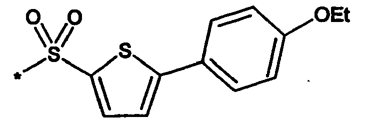 | M+1 : 511 |
| **(as)** | 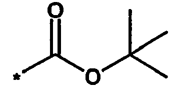 | 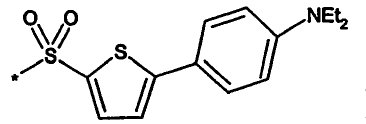 | M+1 : 546.1 |
| **(at) Reference-Example** | 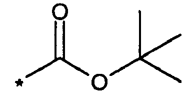 | 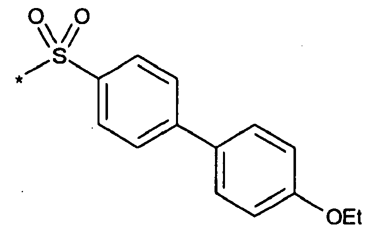 | M+1 : 519.1 |
| **(au)** | 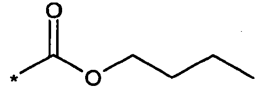 | 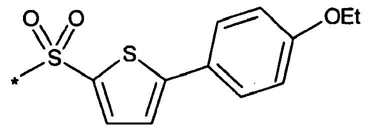 | M-1 : 523.4 |
| **(av)** | 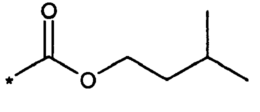 | 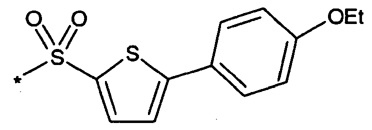 | M-1 : 537.4 |
| **(aw)** | 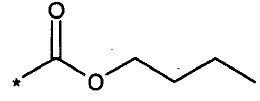 | 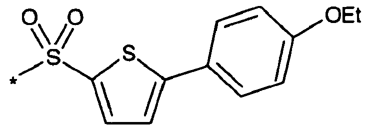 | M-1 : 537.3 |
| **(ax)** | 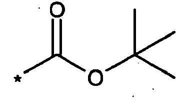 | 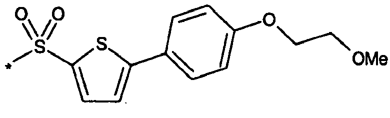 | M-1 : 553.3 |
| **(ay)** | 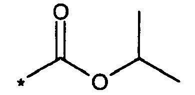 | 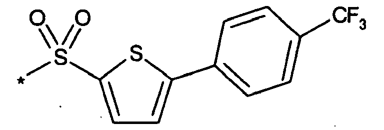 | M-1 : 533.1 |
| **(az)** | 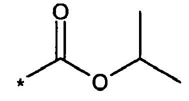 | 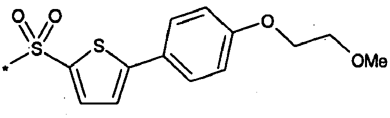 | M-1 : 539.3 |
| **(ba)** | 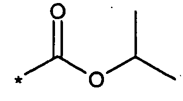 | 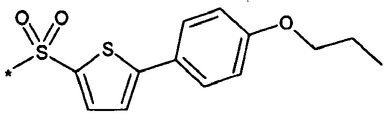 | M-1 : 523.1 |
| **(bb)** | 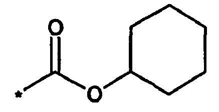 | 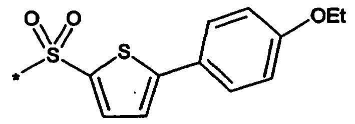 | M+1 : 551 |
| **(bc)** | 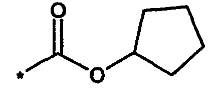 | 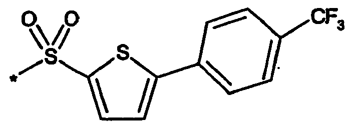 | M-1 : 559.3 |
| **(bd)** | 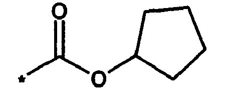 | 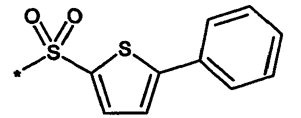 | M-1 : 491.3 |
| **(be)** | 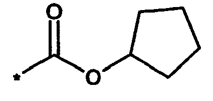 | 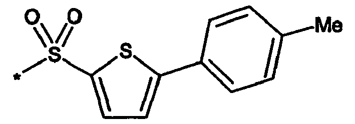 | M-1 : 505.2 |
| **(bf)** | 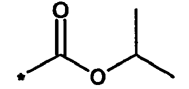 | 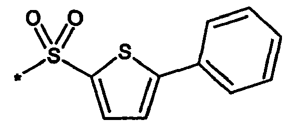 | M-1 : 465.4 |
| **(bg)** | 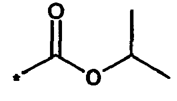 | 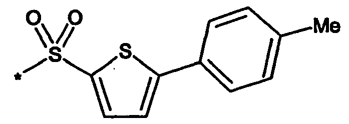 | M-1 : 479.1 |
| **(bh)** | 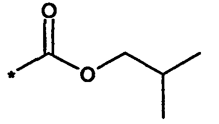 | 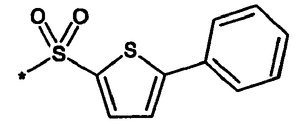 | M-1 : 479.1 |
| **(bi)** | 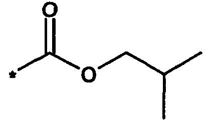 | 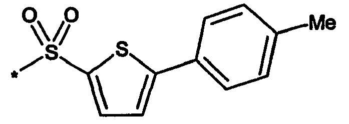 | M-1 : 493.2 |
| **(bj)** | 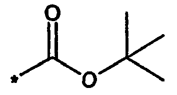 | 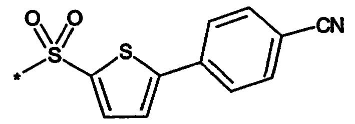 | M-1 : 504.2 |
| **(bk)** | 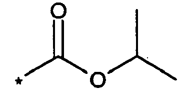 | 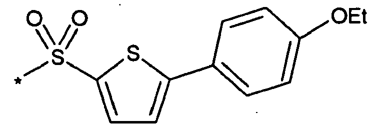 | M+H, M+NH₄: 497.1, 514.2 |
| **(bl)** | 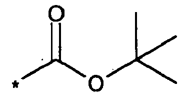 | 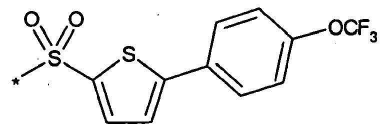 | M+1 : 562.9 |
| **(bm) Reference-Example** | 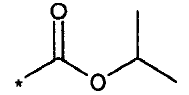 | 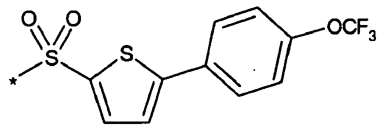 | M+1 : 525.3 M-1 : 523.3 |
| **(bn)** | 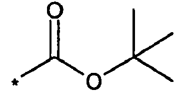 | 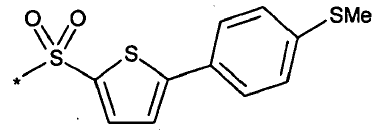 | M-1 : 525.2 |
| **(bo)** | 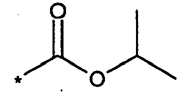 | 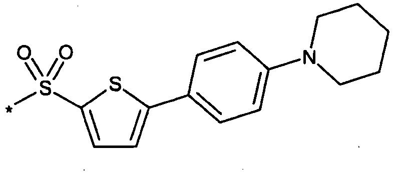 | M+1 : 550.2 |
| **(bp)** | 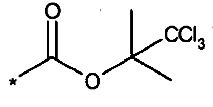 | 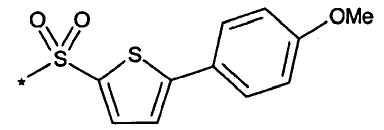 | M-1 : 613.0 |
| **(bq)** | 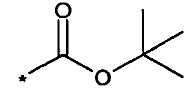 | 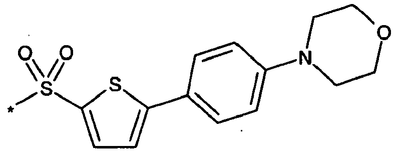 | M+18: 583.3 |
| **(br)** | 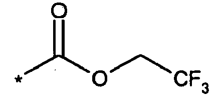 | 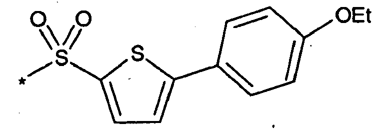 | M-1 : 549.0 |
| **(bs)** | 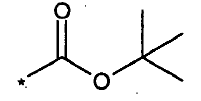 | 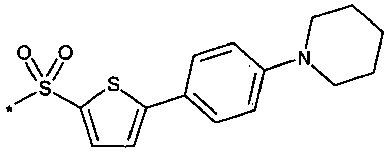 | M+1 : 564.3 |
| **(bt)** | 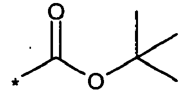 | 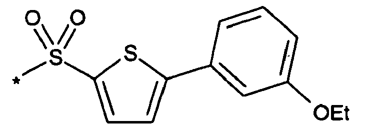 | M-1 : 523.2 |
| **(bu)** | 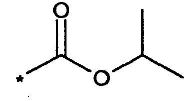 | 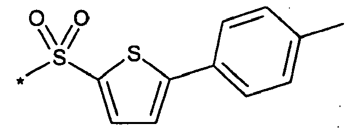 | M-1 : 579.6 |
| **(bv)** | 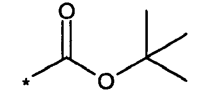 | 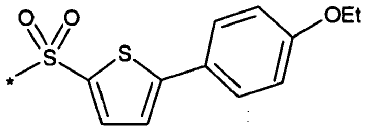 | M-1 : 517.2 |
| **(bw) Reference-Example** | 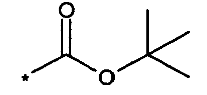 | 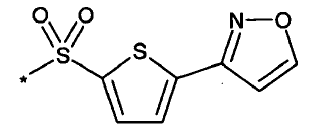 | M+1 : 472.3 |
| **(bx)** | 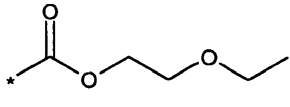 | 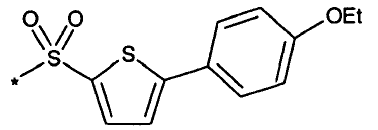 | M+1 : 541.4 |
| **(by)** | 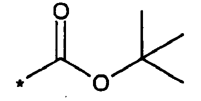 | 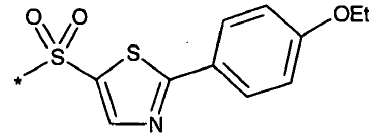 | M-1 : 524.7 |
| **(bz)** | 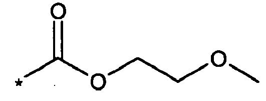 | 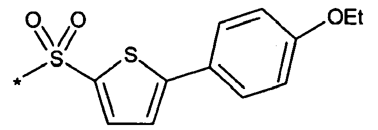 | M+1 : 527.4 |
| **(ca)** | 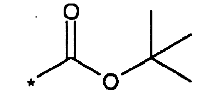 | 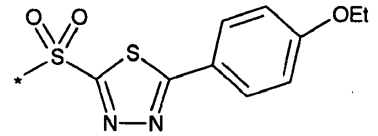 | M-1 : 525.6 |
| **(cb) Reference-Example** | 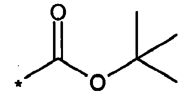 | 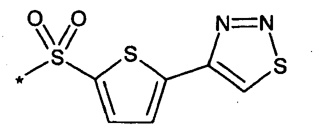 | M11 : 487.6 |
| **(cc) Reference-Example** | 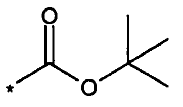 | 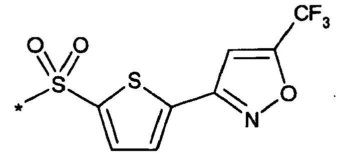 | M-1 : 538.5 |
| **(cd)** | 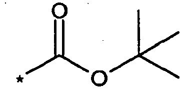 | 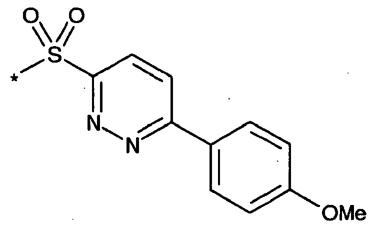 | M-1 : 505.6 |
| **(ce)** | 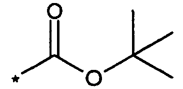 | 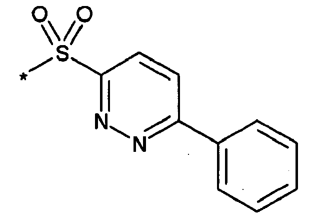 | M-1 : 475.6 |

### Example 7

Prepared by procedures similar to those described in previous examples are the following N-sulfonyl piperidyl derivatives, using the appropriate sulfonyl chloride.

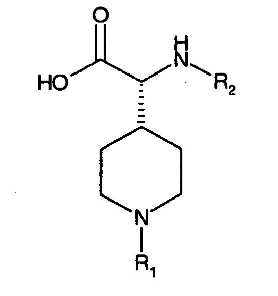

| **Example** | **R₁** | **R₂** | **MS Found** |
|---|---|---|---|
| **(a)** | 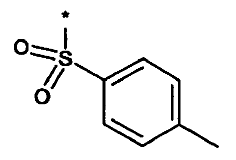 | 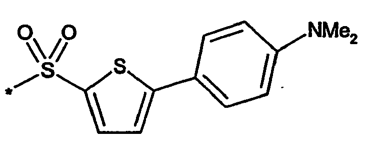 | M+1 : 578.3 |
| **(b)** | 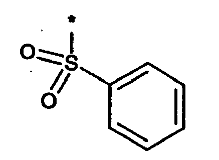 | 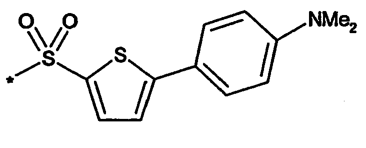 | M+1 : 564.3 |
| **(c)** | 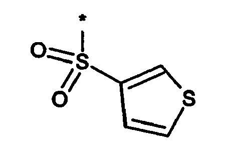 | 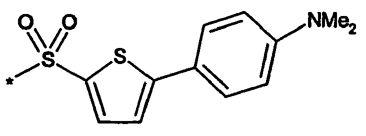 | M+1 : 570.1 |
| **(d)** | 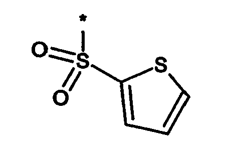 | 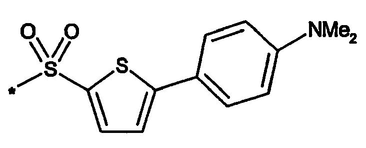 | M+1 : 570.1 |
| **(e)** | 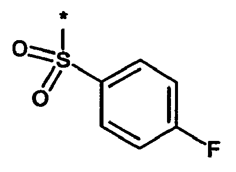 | 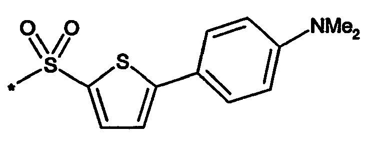 | M+1 : 582.1 |
| **(f)** | 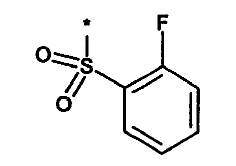 | 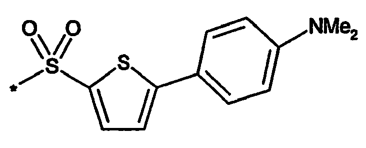 | M+1 : 582.1 |
| **(g)** | 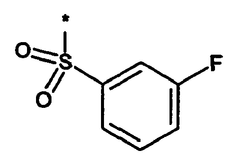 | 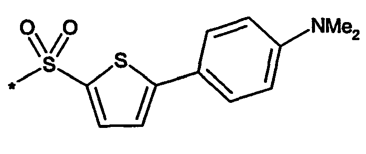 | M+H : 582.1 |
| **(h)** | 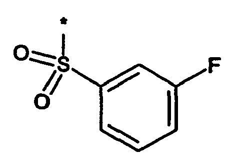 | 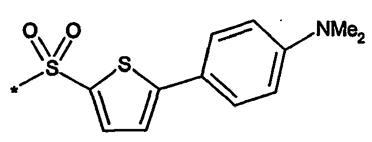 | M+1 : 596.1 |
| **(i)** | 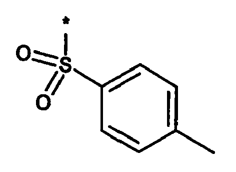 | 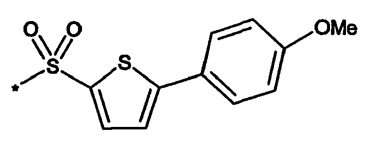 | M+1 : 565.3 |
| **(j)** | 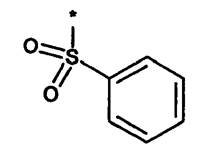 | 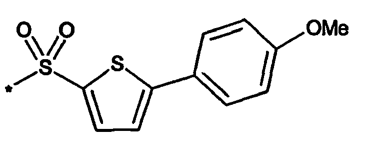 | M+1 : 551.3 |
| **(k)** | 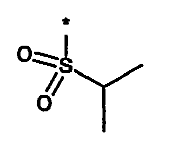 | 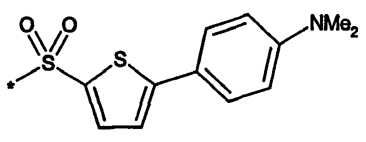 | M+1, M+NH4 530.2, 547.2 |
| **(l)** | 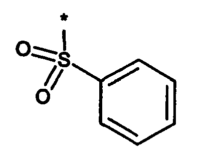 | 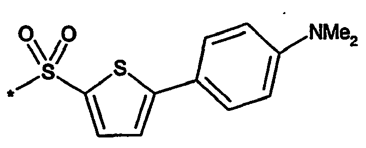 | M+1 : 564.1 |
| **(m)** | 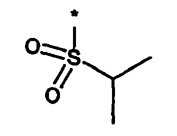 | 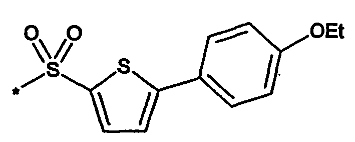 | M-1 : 529.1 |
| **(n)** | 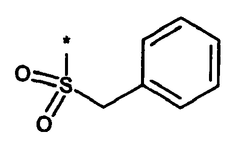 | 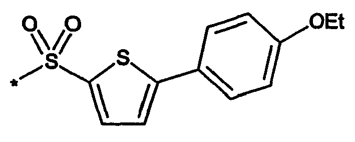 | M-1 : 577.1 |
| **(o)** | 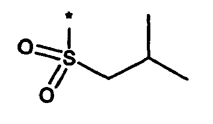 | 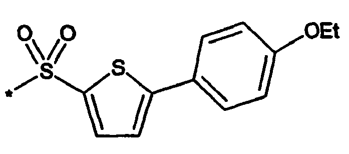 | M-1 : 543.3 |
| **(p)** | 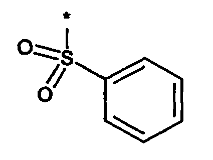 | 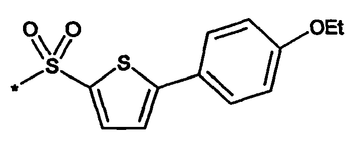 | M-1 : 563.1 |
| **(q)** | 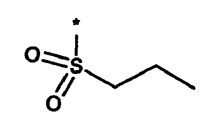 | 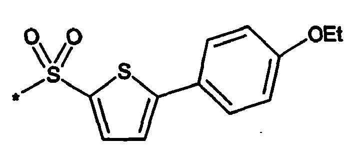 | M-1 : 529 |
| **(r)** | 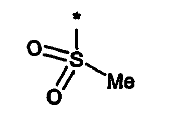 | 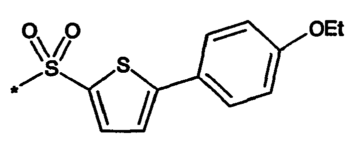 | M-1 : 501 |
| **(s)** | 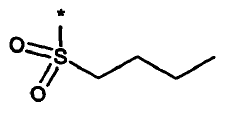 | 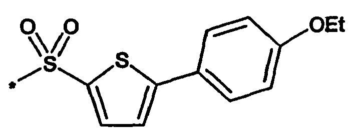 | M-1 : 543 |
| **(t)** | 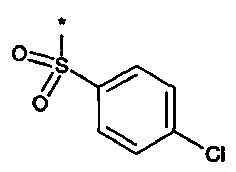 | 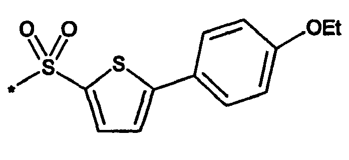 | M-1 : 597.1 |
| **(u)** | 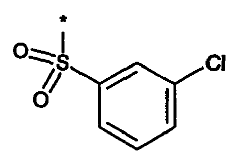 | 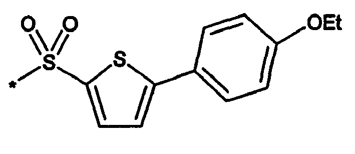 | M-1 : 597.4 |

### Example 8

Prepared by procedures similar to those described in Example 4 are the following areas, using the appropriate isocyanate or carbamoyl chloride.

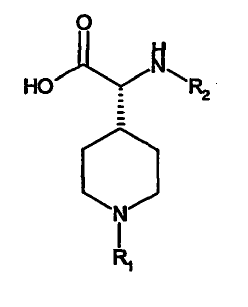

| **Example** | **R₁** | **R₂** | **MS Found** |
|---|---|---|---|
| **(a)** | 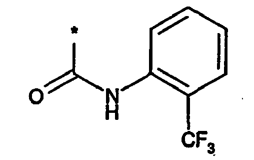 | 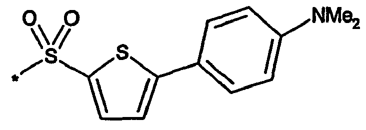 | M+1 : 611.4 |
| **(b)** | 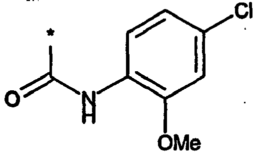 | 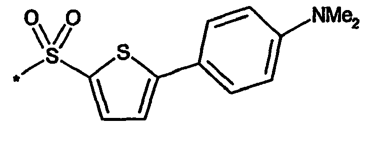 | M+1 : 607.3 |
| **(c)** | 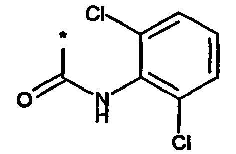 | 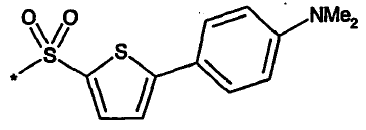 | M+1 : 611.3 |
| **(d)** | 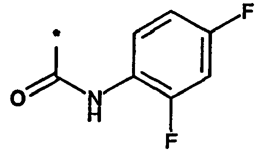 | 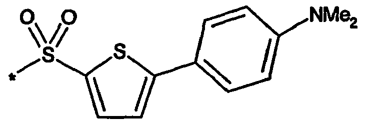 | M+1 : 579.4 |
| **(e)** | 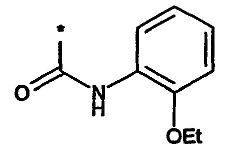 | 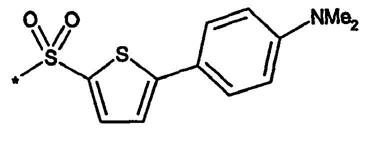 | M+1 : 587.4 |
| **(f)** | 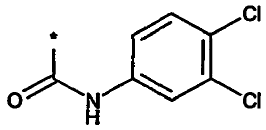 | 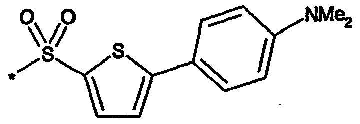 | M+1 : 611.3 |
| **(g)** | 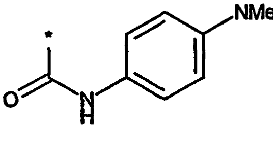 | 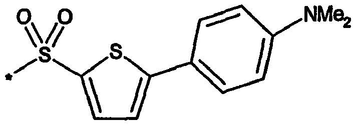 | M+1 : 586.4 |
| **(h)** | 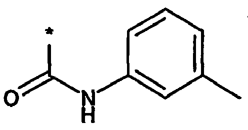 | 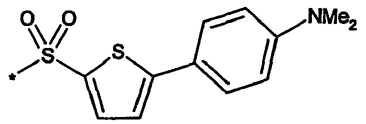 | M+1 : 557.4 |
| **(i)** | 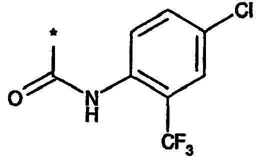 | 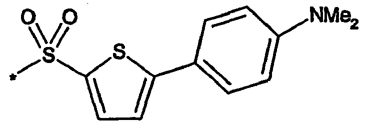 | M+1 : 645.3 |
| **0)** | 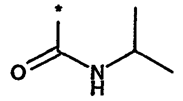 | 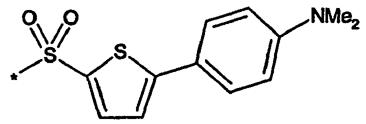 | M+1 : 509.2 |
| **(k)** | 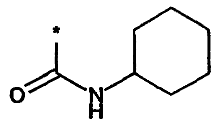 | 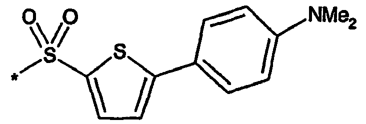 | M+1 : 549.2 |
| **(l)** | 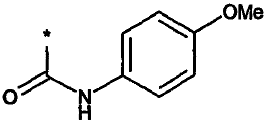 | 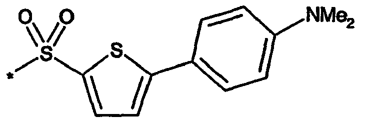 | M+1 573.2 |
| **(m)** | 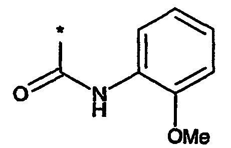 | 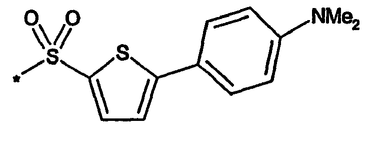 | M+1 : 573.2 |
| **(n)** | 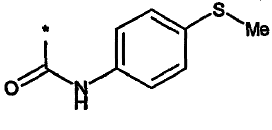 | 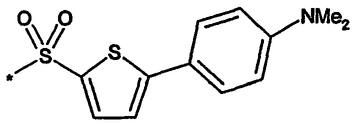 | M+1 : 589.1 |
| **(o)** | 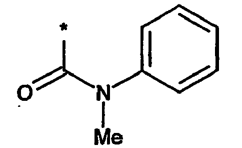 | 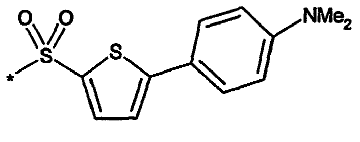 | M+1 : 557.2 |
| **(p)** | 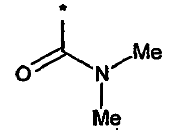 | 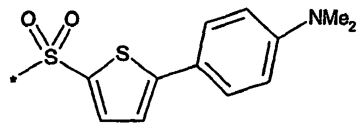 | M+1 : 495.2 |
| **(q)** | 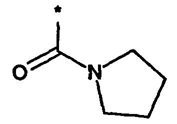 | 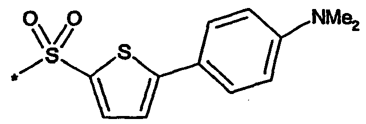 | M+1 : 521.2 |
| **(r)** | 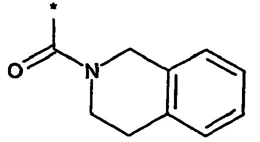 | 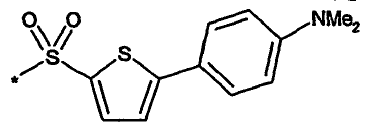 | M+1 : 583.1 |
| **(s)** | 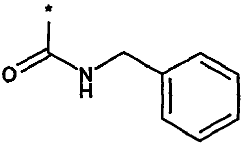 | 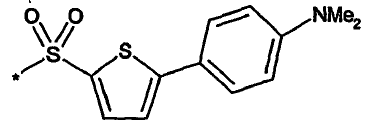 | M+1 : 557.2 |
| **(t)** | 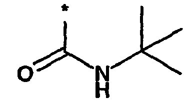 | 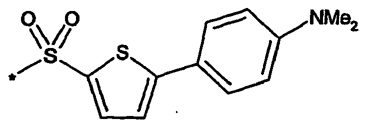 | M+1 : 523.2 |
| **(u)** | 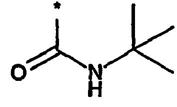 | 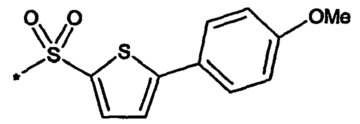 | M+1 : 510.2 |
| **(v)** | 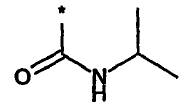 | 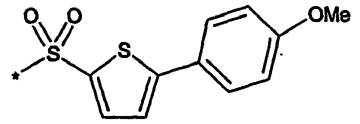 | M+1 : 496.4 |
| **(w)** | 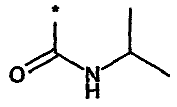 | 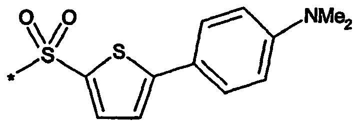 | M+1 : 509.19 |
| **(x)** | 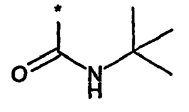 | 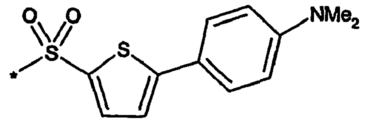 | M+1 : 523.1 |
| **(y)** | 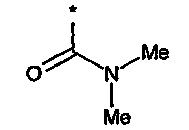 | 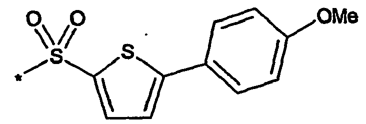 | M+1 : 482.2 |
| **(z)** | 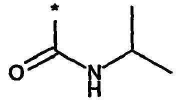 | 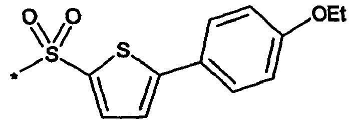 | M+1 : 510.2 |
| **(aa)** | 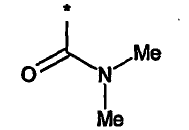 | 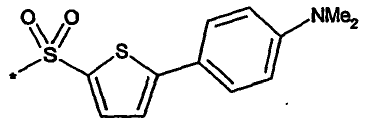 | M+1 : 495.2 |
| **(ab)** | 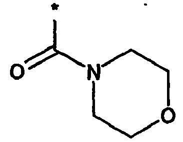 | 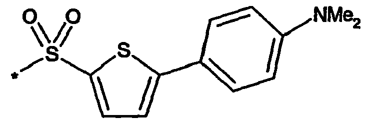 | M+1 : 537.2 |
| **(ac)** | 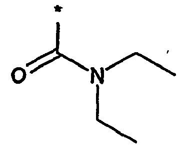 | 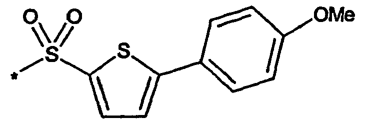 | M+1 : 510.3 |
| **(ad)** | 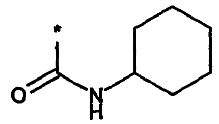 | 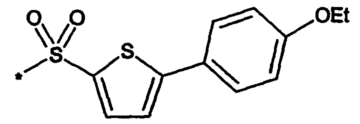 | M+1 : 550.4 |
| **(ae)** | 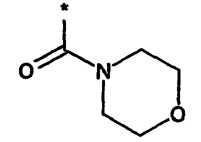 | 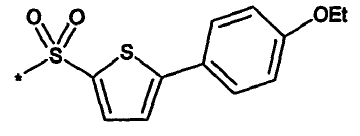 | M+1 : 538.4 |

### Example 9

(α*R*)-N-BOC-α-[[[5-(4-ethoxyphenyl)-2-thienyl]sulfonyl]-amino]-4-piperidineacetic acid (150 mg, 0.29 mmol) is added to a suspension of cesium carbonate (48 mg, 0.15 mmol) in dry DMF (10 mL) at room temperature. Benzyl bromide (49 mg, 0.29 mmol) is added to the mixture and the reaction is stirred at room temperature for two hours. The reaction mixture is diluted with water and extracted with EtOAc. The combined organic extracts are washed with LiCl (sat. aq.), brine, dried over Na₂SO₄ and evaporated *in vacuo* to give (α*R*)-1-BOC-α-[[[5-(4-ethoxyphenyl)-2-thienyl]sulfonyl]-amino]-4-piperidineacetic acid benzyl ester.

To a suspension of K₂CO₃ in dry DMF (15 mL) at room temperature is added (α*R*)-1-BOC-α-[[[5-(4-ethoxyphenyl)-2-thienyl]sulfonyl]amino]-4-piperidineacetic acid benzyl ester (0.58 mmol) and methyl iodide (0.61 mmol). The reaction mixture is stirred at room temperature for 3 hours after which the mixture is diluted with water and extracted with EtOAc. The combined organic layers are combined and washed with 1 N LiCl (aq.) and brine, then dried over Na₂SO₄ and evaporated *in vacuo* to yield (α*R*)-1-BOC-α-[[[5-(4-ethoxyphenyl)-2-thienyl]sulfonyl]-N-methyl-amino]-4-piperidineacetic acid benzyl ester as a yellow oil.

A mixture of (α*R*)-1-BOC-α-[[[5-(4-ethoxyphenyl)-2-thienyl]sulfonyl]-N-methyl-amino]-4-piperidineacetic acid benzyl ester (330 mg) and 10% Pd/C (160 mg) in EtOH (20 mL) is placed under a 50 psi pressure of H₂ (g) in a Parr apparatus for 18 hours. The reaction mixture is then filtered through celite and the filtrate is concentrated *in vacuo*. The product is purified by flash chromatography on silica gel using a gradient elution of 2-5% MeOH/CH₂Cl₂. Evaporation of the solvents yields (α*R*)-1-BOC-α-[[[5-(4-ethoxyphenyl)-2-thienyl]sulfonyl]-N-methyl-amino]-4-piperidineacetic acid as a white foam, MS (M-1 : 517.3), having the following structure:

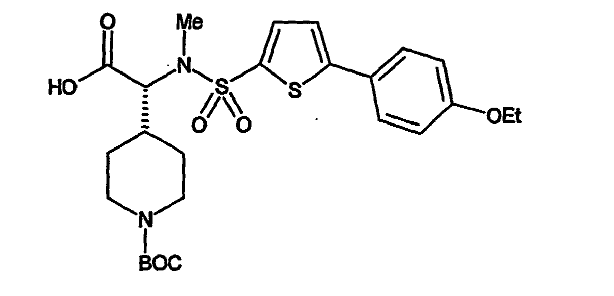

Similarly prepared are:
(a) (αR)-1-[(1-methylethoxy)carbonyl]-α-[[[5-(4-ethoxyphenyl)-2-thienyl]sulfonyl]-N-methyl-amino]-4-piperidineacetic acid; MS (M+1) : 525.3, (M-1) : 523.3.
(b) (αR)-1-[(1-methylethoxy)carbonyl]-α-[[[5-(4-ethoxyphenyl)-2-thienyl]sulfonyl]-N-(propyl)-amino]-4-piperidineacetic acid; MS (M+1): 553.1, (M-1) : 551.0.
(c) (αR)-1-[(cyclopentyloxy)carbonyl]-α-[[[5-(4-ethoxyphenyl)-2-thienyl]sulfonyl]-N-methyl-amino]-4-piperidineacetic acid; MS (M+1): 551.3, (M-1) : 549.3.
(d) (αR)-1-BOC-α-[[[4-(4-methoxyphenyl)-phenyl]sulfonyl]-N-methyl-amino]-4-piperidineacetic acid; MS (M+1) 519.4: (M-1) : 517.4.

### Reference-Example 10

A mixture of 0.26 g (0.5 mmol) of (αR-1-BOC-α-[[[5-(4-ethoxyphenyl)-2-thienyl]sulfonyl]amino]-4-piperidineacetic in 1 mL of 48% HBr under an atmosphere of nitrogen is heated to 100°C for 5 hours. The reaction mixture is cooled to room temperature and stirred overnight. The solvent is evaporated in vacuo to give (αR)-α-[[[5-(4-hydroxyphenyl)-2-thienyl]sulfonyl]amino]-4-piperidineacetic acid as a solid.

### Example 11

To a solution of 0.25 g (0.52 mmol) αR)-α-[[[5-(4-hydroxyphenyl)-2-thienyl]sulfonyl]amino]-4-piperidineacetic acid in 15 mL of THF cooled to 0°C is added 0.78 mL (3.14 mmol) of *N,O-bis*-trimethylsilylacetamide. This is followed by the addition of 1.05 mL (1.05 mmol) of a 1 M toluene solution of isopropyl chloroformate. The mixture is stirred under an atmosphere of nitrogen and allowed to warm to room temperature overnight. The mixture is treated with 5 mL of a saturated aqueous solution of NaHCO₃, and then extracted 2 times with 20 mL of diethyl ether. The aqueous layer is acidified to pH 4 with 1 N HCl, and extracted 3 times with 30 mL of ethyl acetate.

The organic layers are washed with saturated brine (30 mL), dried over magnesium sulfate, and filtered. The solvent is removed *in vacuo* and the residue purified by column chromatography (LiChroprep®DIOL, 3% MeOH/CH₂Cl₂) to give (αR)-1-[(1-methylethoxy)carbonyl]-α[[[5-(4-hydroxyphenyl)-2-thienyl]sulfonyl]amino-4-piperidineacetic acid, m.p. 113-115°C.

### Reference-Example 12

A solution of (αR)-1-BOC-α-[[[5-(4-methoxyphenyl)-2-thienyl]sulfonyl]amino]-4-piperidineacetic acid (1 mmol), HOAT (1 mmol), EDCl (1.3 mmol), O-(2-tetrahydropyranyl)-hydroxylamine (5 mmol) and excess N-methylmorpholine (2 mL) in CH₂Cl₂ (15 mL) is stirred overnight at room temperature. Water is added and the layers separated. The aqueous layer is further washed with CH₂Cl₂ and the combined organic layers dried over magnesium sulfate. Filtration and the removal of solvent *in vacuo* gives material for use in the next step. The material is combined with acetic acid (10 mL), water (3 mL) and THF (4 mL) and heated for 7 hours at 75°C. The mixture is concentrated *in vacuo,* water and ethyl acetate added and the layers separated. The aqueous layers are combined and washed with ethyl acetate and the combined organic layers washed with a saturated sodium chloride solution. The organic layer is dried over magnesium sulfate, filtered and solvent removed *in vacuo.* The crude residue is purified via column chromatography (ethyl acetate:hexane, 2:1,then ethyl acetate on silica). The product fractions are combined and the solvent removed *in vacuo.* The resulting white foam is recrystallized from CH₂Cl₂-hexane to give white crystals of (αR)-1-BOC-α-[[[5-(4-methoxyphenyl)-2-thienyl]sulfonyl]amino]-4-piperidine-(N-hydroxy)-acetamide.

Similarly prepared are:
(a) (αR)-1-BOC-α-[[[4-(4-methoxyphenyl)-phenyl]sulfonyl]amino]-4-piperidine-(N-hydroxy)-acetamide (Reference-Example).
(b) (αR)-1-BOC-α-[[[5-(4-methoxyphenyl))-2-thienyl]sulfonyl]amino]-4-piperidine-(N-hydroxy)-acetamide (Reference-Example).
(c) (αR)-1-[(cyclopentyloxy)carbonyl]-α-[[[5-(4-ethoxyphenyl)-2-thienyl]sulfonyl]amino]-4-piperidine-(N-hydroxy)-acetamide (Reference-Example).

### Reference-Example 13

(a) To a solution of (αR)-1-[(2-phenylethoxy)-carbonyl]-α-[[[4-(4-chlorophenyl)phenyl]sulfonyl]amino]-4-piperidineacetic acid (91 mg, 0.16 mmol) in methylene chloride (3 mL) is added O-tritylhydroxylamine (135 mg, 0.49 mmol), N-methylmorpholine (0.053 mL, 0.49 mmol), 1-hydroxy-7-azabenzotriazole (40 mg, 0.16 mmol) and 1-ethyl-3-(3-dimethylaminopropyl)-carbodiimide hydrochloride. The reaction mixture is stirred at room temperature for 18 hours, diluted with ethyl acetate and washed with water. The aqueous layer is extracted with methylene chloride, the combined organic layers are washed with 1 N aqueous hydrochloric acid, saturated aqueous sodium bicarbonate, brine, dried over sodium sulfate, and concentrated in vacuo. The crude product is purified by silica gel chromatography (10% to 40% ethyl acetate in hexanes) to provide (αR)-1-[(2-phenylethoxy)carbonyl]-α-[[[4-(4-chlorophenyl)-phenyl]sulfonyl]amino]-4-piperidine-(N-trityloxy)-acetamide.

To a solution of (αR)-1-[(2-phenylethoxy)carbonyl]-α-[[[4-(4-chlorophenyl)phenyl]sulfonyl]amino]-4-piperidine-(N-trityloxy)-acetamide (86 mg, 0.105 mmol) in methylene chloride (5 mL) cooled to 0°C is added triethyl silane (0;033 mL, 0.21 mmol) followed by slow addition of trifluoroacetic acid (0.032 mL, 0.423 mmol). After stirring at 0°C for 5 minutes, the solvent is removed *in vacuo,* at room temperature. The resulting solid is dried under high vacuum and dissolved in the minimum amount of methylene chloride (ca. 2.5 mL). A mixture of diethyl ether (ca. 5 mL) and pentane (ca. 2.5 mL) is slowly added and the resulting white precipitate is triturated, filtered and washed with pentane (Note: this precipitation process is repeated a second time if high purity is not obtained after the first attempt), to provide (αR)-1-[(2-phenylethoxy)carbonyl]-α-[[[4-(4-chlorophenyl)phenyl]sulfonyl]amino]-4-piperidine-(N-hydroxy)-acetamide; m.p. 200°C; [M+1] = 572.

The acid can be prepared similarly to procedures described hereinabove; and also similarly to Example 1 of U.S. Patent No. 5,817,822, namely as follows using 4-(4-chlorophenyl)benzenesulfonyl chloride instead of 4-methoxybenzenesulfonyl chloride:
(R)-(1-BOC-4-piperidinyl)-glycine (see U.S. Patent No. 5,817,822) is condensed with 4-(4-chlorophenyl)-benzenesulfonyl chloride to give (αR)-1-BOC-α-[[[4-(4-chlorophenyl)phenyl]sulfonyl]amino]-4-piperidineacetic acid which is in turn converted to benzyl (αR)-1-BOC-α-[[[4-(4-chlorophenyl)-phenyl]sulfonyl]amino]-4-piperidine acetate. Reaction with (2-pyridyl) (phenylethyl)-carbonate (prepared in situ according to Ghosh, Tetrahedron Letters, Vol. 32, pp. 4251-4254 (1991)) yields benzyl (αR)-1-[(2-phenylethoxy)-carbonyl]-α-[[[4-(4-chlorophenyl)-phenyl]sulfonyl]amino]-4-piperidine acetate which is in turn hydrogenolyzed to yield (αR)-1-[(2-phenylethoxy)-carbonyl]-α-[[[[4-(4-chlorophenyl)phenyl]sulfonyl]amino]-4-piperidineacetic acid.

(b) Similarly prepared is (αR)-1-[[2-(1-naphthyl)ethoxy]-carbonyl]-α-[[[4-(4-chlorophenyl)phenyl]sulfonyl]amino]-4-piperidine-(N-hydroxy)-acetamide; m.p.180-182°C; [M+NH₄] = 639.

## Claims

1. A compound of the formula 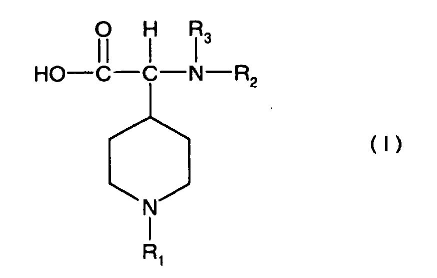
R₁ represents acyl derived from a carboxylic acid, a carbonic acid, a carbamic acid or a sulfonic acid;
R₂ represents biarylsulfonyl in which biaryl represents thienyl or furanyl substituted by phenyl which is optionally substituted by radicals selected from C₁-C₇alkyl, C₁-C₄alkoxy, C₁-C₇alkyl-(thio, sulfinyl or sulfonyl), C₁-C₄alkoxy-C₁-C₄alkoxy, hydroxy, halogen, cyano, trifluoromethyl, amino, mono- or di-C₁-C₇alkylamino, heterocyclyl which is piperidinyl, pyrrolidinyl, morpholinyl, piperazinyl, N-(C₁-C₇alkyl or aryl-C₁-C₇alkyl)-piperazinyl wherein aryl is phenyl; perhydrofuranyl, perhydropyranyl or tetrahydroisoquinolyl, and from C₁-C₇-alkylenedioxy;
R₃ represents hydrogen or C₁-C₇ alkyl;
a pharmaceutically acceptable prodrug ester derivative thereof; or a pharmaceutically acceptable salt thereof.

2. A compound of the formula III 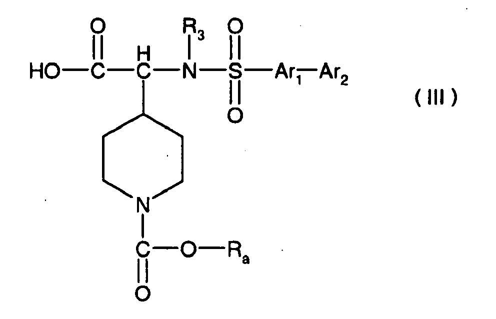 wherein
R₃ represents hydrogen or C₁-C₇ alkyl;
Ar₁ represents thiazolylene, thiadiazolylene or pyridazinylene;
Ar₂ represents monocyclic carbocyclic aryl, or optionally substituted pyridyl; or Ar₂ represents optionally substituted isoxazolyl or optionally substituted thiadiazolyl;
said optional substituents being selected from the group consisting of optionally substituted C₁-C₇ alkyl, C₁-C₄ alkoxy, cyano, hydroxy, acyl, trifluoro- C₁-C₄ alkoxy, trifluoromethyl or halogen;
wherein the optional substituent or substitutents on the C₁-C₇ alkyl are selected from halo, trihalomethyl, hydroxy, alkoxy, alkoxyalkoxy, aryloxy, cycloalkyl, alkanoyl, alkanoyloxy, amino, substituted amino, alkanoylamino, thiol, alkylthio, arylthio, alkylthiono, alkylsulfonyl, arylsulfonyl, aminosulfonyl, nitro, cyano, carboxy, carbamyl, alkoxycarbonyl, aralkoxy or heterocyclyl; where aryl where mentioned is phenyl and heterocyclyl is piperidinyl, pyrrolidinyl, morpholinyl, piperazinyl, N-(C₁-C₇alkyl or aryl-C₁-C₇alkyl)-piperazinyl wherein aryl is phenyl; perhydrofuranyl, perhydropyranyl or tetrahydroisoquinolyl;
and Rₐ represents C₁-C₇ alkyl or cycloalkyl; pharmaceutically acceptable prodrug ester derivatives thereof; or a pharmaceutically acceptable salt thereof.

3. A compound according to claim 2 of the formula IIIa 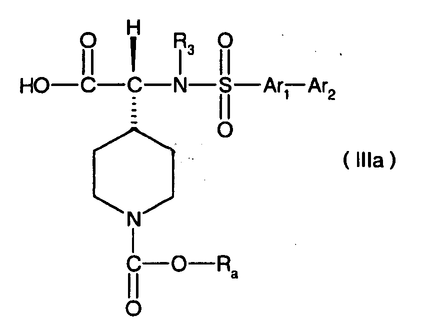 wherein R₃, Rₐ, Ar₁ and Ar₂ have meaning as defined in said claim; a pharmaceutically acceptable prodrug ester derivative thereof, or a pharmaceutically acceptable salt thereof.

4. A pharmaceutical composition comprising a compound according to any one of the preceding claims in combination with one or more pharmaceutically acceptable carriers.)

5. A compound according to any one of claims 1 to 3 for use in inhibiting MMP-13 in a mammal.

6. A compound according to any one of claims 1 to 3 for use in treating inflammatory conditions in a mammal.

7. Use of a compound according to any one of claims 1 to 3 in the manufacture of a medicament for inhibiting MMP-13 in a mammal.

8. Use of a compound according to any one of claims 1 to 3 in the manufacture of a medicament for treating inflammatory conditions in a mammal.

## Patentansprüche

1. Verbindung der Formel 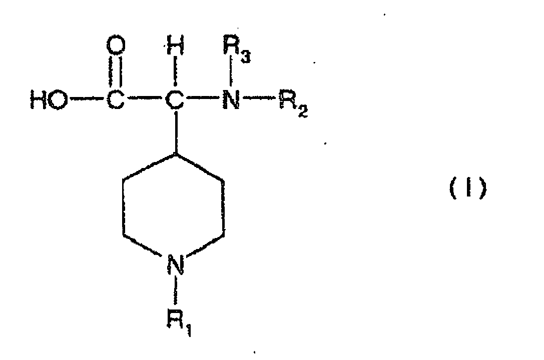 worin
R₁ für von einer Carbonsäure, einer Kohlensäure, einer Carbamidsäure oder einer Sulfonsäure abgeleitetes Acyl steht;
R² für Biarylsulfonyl steht, das für durch Phenyl substituiertes Thienyl oder Furanyl, das gegebenenfalls durch Reste, die unter C₁-C₇-Alkyl, C₁-C₄-Alkoxy, C₁-C₇-Alkylthio, C₁-C₇-Alkylsulfinyl, C₁-C₇-Alkylsulfonyl, C₁-C₄-Alkoxy-C₁-C₄-alkoxy, Hydroxy, Halogen, Cyano, Trifluormethyl, Amino, Mono- oder Di-C₁-C₇-alkylamino, Heterocyclyl, bei dem es sich um Piperidinyl, Pyyrolidinyl, Morpholinyl, Piperazinyl, N-(C₁-C₇-Alkyl)-piperazinyl, N-(Aryl-C₁-C₇-alkyl)piperazinyl, worin Aryl für Phenyl steht, Perhydrofuranyl, Perhydropyranyl oder Tetrahydroisochinolinyl handelt, und C₁-C₇-Alkylendioxy ausgewählt sind, substituiert ist, steht und
R₃ für Wasserstoff oder C₁-C₇-Alkyl steht;
ein pharmazeutisch unbedenkliches Prodrug-Esterderivat davon oder ein pharmazeutisch unbedenkliches Salz davon.

2. Verbindung der Formel III 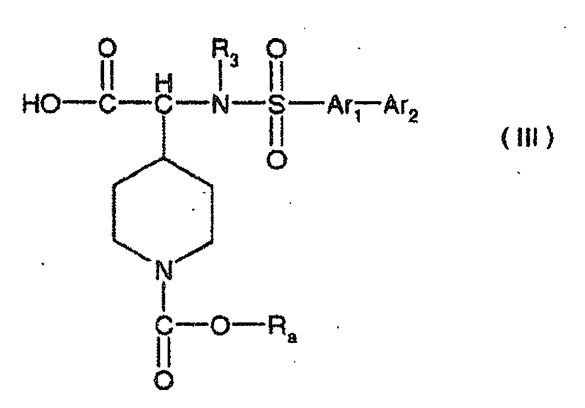 worin
R₃ für Wasserstoff oder C₁-C₇-Alkyl steht;
Ar₁ für Thiazolylen, Thiadiazolylen oder Pyridazinylen steht;
Ar₂ für monocyclisches carbocyclisches Aryl oder gegebenenfalls substituiertes Pyridyl steht oder Ar₂ für gegebenenfalls substituiertes Isoxazolyl oder gegebenenfalls substituiertes Thiadiazolyl steht;
wobei die fakultativen Substituenten aus der Gruppe bestehend aus gegebenenfalls substituiertem C₁-C₇-Alkyl, C₁-C₄-Alkoxy, Cyano, Hydroxy, Acyl, Trifluor-C₁-C₄-alkoxy, Trifluormethyl oder Halogen ausgewählt sind;
wobei der oder die fakultativen Substituenten an dem C₁-C₇-Alkyl unter Halogen, Trihalogenmethyl, Hydroxy, Alkoxy, Alkoxyalkoxy, Aryloxy, Cycloalkyl, Alkanoyl, Alkanoyloxy, Amino, substituiertem Amino, Alkanoylamino, Thiol, Alkylthio, Arylthio, Alkylthiono, Alkylsulfonyl, Arylsulfonyl, Aminosulfonyl, Nitro, Cyano, Carboxy, Carbamyl, Alkoxycarbonyl, Aralkoxy oder Heterocyclyl ausgewählt sind; wobei Aryl, wo erwähnt, für Phenyl steht und Heterocyclyl für Piperidinyl, Pyrrolidinyl, Morpholinyl, Piperazinyl, N-(C₁-C₇-Alkyl)piperazinyl, N-(Aryl-C₁-C₇-alkyl)piperazinyl, worin Aryl für Phenyl steht, Perhydrofuranyl, Perhydropyranyl oder Tetrahydroisochinolinyl steht;
und Rₐ für C₁-C₇-Alkyl oder Cycloalkyl steht;
pharmazeutisch unbedenklichee Prodrug-Esterderivate davon oder ein pharmazeutisch unbedenkliches Salz davon.

3. Verbindung nach Anspruch 2 der Formel IIIa 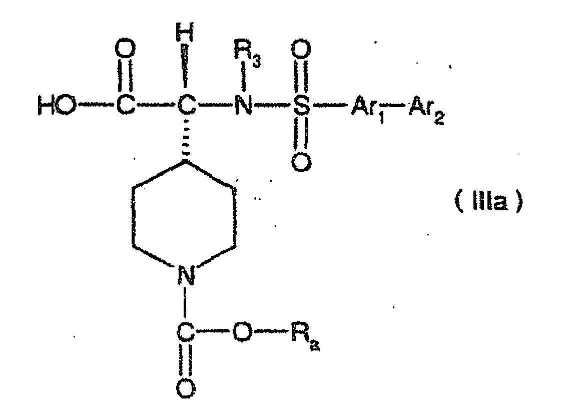
worin R₃, Rₐ, Ar₁ und Ar₂ die in dem Anspruch angegebene Bedeutung besitzen;
ein pharmazeutisch unbedenkliches Prodrug-Esterderivat davon oder ein pharmazeutisch unbedenkliches Salz davon.

4. Pharmazeutische Zusammensetzung, enthaltend eine Verbindung nach einem der vorhergehenden Ansprüche in Kombination mit einem oder mehreren pharmazeutisch unbedenklichen Trägern.

5. Verbindung nach einem der Ansprüche 1 bis 3 zur Verwendung bei der Inhibierung von MMP-13 bei einem Säugetier.

6. Verbindung nach einem der Ansprüche 1 bis 3 zur Verwendung bei der Behandlung von entzündlichen Zuständen bei einem Säugetier.

7. Verbindung nach einem der Ansprüche 1 bis 3 zur Verwendung bei der Herstellung eines Arzneimittels zur Inhibierung von MMP-13 bei einem Säugetier.

8. Verbindung nach einem der Ansprüche 1 bis 3 zur Verwendung bei der Herstellung eines Arzneimittels zur Behandlung von entzündlichen Zuständen bei einem Säugetier.

## Revendications

1. Composé représenté par la formule 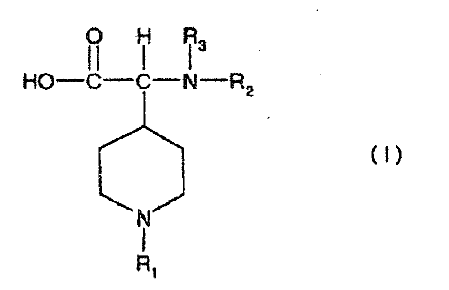 dans laquelle
R₁ représente un acyle dérivé d'un acide carboxylique, d'un acide carbonique, d'un acide carbamique ou d'un acide sulfonique ;
R₂ représente un biarylsulfonyle dans lequel le biaryle représente un thiényle ou furanyle substitué par un phényle qui est facultativement substitué par des radicaux choisis parmi les radicaux alkyle en C₁-C₇, alcoxy en C₁-C₄, (alkyl en C₁-C₇)(thio, sulfinyle ou sulfonyle), (alcoxy en C₁-C₄) (alcoxy en C₁-C₄), hydroxy, halogéno, cyano, trifluorométhyle, amino, mono- ou di (alkyl en C₁-C₇)amino, hétérocyclyle qui est un pipéridinyle, pyrrolidinyle, morpholinyle, pipérazinyle, *N*-(alkyl en C₁-C₇, ou aryl- (alkyl en C₁-C₇))-pipérazinyle où l'aryle est un phényle ; perhydrofuranyle, perhydropyranyle ou tétrahydroisoquinolyle et parmi les radicaux (alkylène en C₁-C₇) dioxy ;
R₃ représente l'hydrogène ou un alkyle en C₁-C₇ ;
dérivé ester promédicament pharmaceutiquement acceptable de celui-ci ; ou sel pharmaceutiquement acceptable de celui-ci.

2. Composé représenté par la formule III 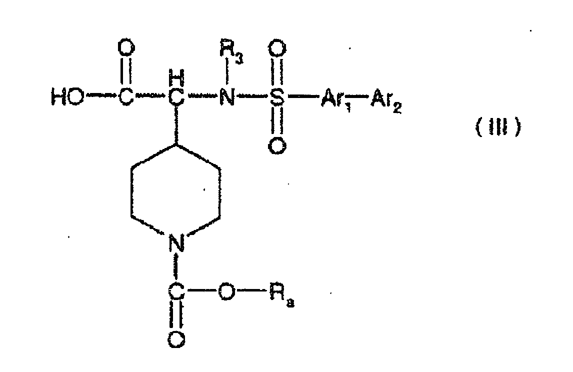 dans laquelle
R₃ représente l'hydrogène ou un alkyle en C₁-C₇ ;
Ar₁ représente un thiazolylène, thiadiazolylène ou pyridazinylène ;
Ar₂ représente un aryle carbocyclique monocyclique ou un pyridyle facultativement substitué ; ou Ar₂ représente un isoxazolyle facultativement substitué ou thiadiazolyle facultativement substitué ;
lesdits substituants facultatifs étant choisis dans le groupe constitué par les substituants alkyle en C₁-C₇ facultativement substitué, alcoxy en C₁-C₄, cyano, hydroxy, acyle, trifluoro (alcoxy en C₁-C₄), trifluorométhyle ou halogéno ;
le substituant ou les substituants facultatifs sur l'alkyle en C₁-C₇ étant choisis parmi les substituants halogéno, trihalogénométhyle, hydroxy, alcoxy, alcoxyalcoxy, aryloxy, cycloalkyle, alcanoyle, alcanoyloxy, amino, amino substitué, alcanoylamino, thiol, alkylthio, arylthio, alkylthiono, alkylsulfonyle, arylsulfonyle, aminosulfonyle, nitro, cyano, carboxy, carbamyle, alcoxycarbonyle, aralcoxy ou hétérocyclyle ; où l'aryle quand il est mentionné est un phényle et l'hétérocyclyle est un pipéridinyle, pyrrolidinyle, morpholinyle, pipérazinyle, *N*-(alkyl en C₁-C₇ ou aryl-(alkyl en C₁-C₇))-pipérazinyle où l'aryle est un phényle ; perhydrofuranyle, perhydropyranyle ou tétrahydroisoquinolyle
et Rₐ représente un alkyle en C₁-C₇ ou un cycloalkyle ;
dérivés esters promédicaments pharmaceutiquement acceptables de celui-ci ; ou sel pharmaceutiquement acceptable de celui-ci.

3. Composé selon la revendication 2 représenté par la formule IIIa 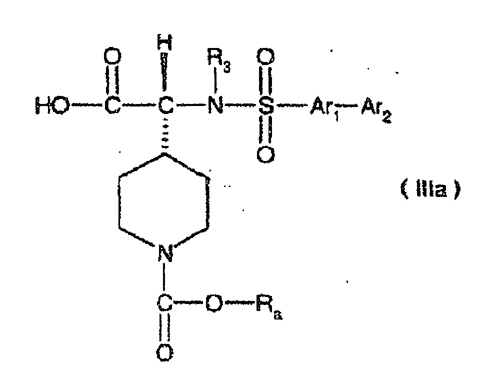 dans laquelle R₃, Rₐ, Ar₁ et Ar₂ ont la signification telle que définie dans ladite revendication ; dérivé ester promédicament pharmaceutiquement acceptable de celui-ci ; ou sel pharmaceutiquement acceptable de celui-ci.

4. Composition pharmaceutique comprenant un composé selon l'une quelconque des revendications précédentes en association avec un ou plusieurs véhicules pharmaceutiquement acceptables.

5. Composé selon l'une quelconque des revendications 1 à 3 destiné à être utilisé dans l'inhibition de la MMP-13 chez un mammifère.

6. Composé selon l'une quelconque des revendications 1 à 3 destiné à être utilisé dans le traitement d'affections inflammatoires chez un mammifère.

7. Utilisation d'un composé selon l'une quelconque des revendications 1 à 3 dans la fabrication d'un médicament pour l'inhibition de la MMP-13 chez un mammifère.

8. Utilisation d'un composé selon l'une quelconque des revendications 1 à 3 dans la fabrication d'un médicament pour le traitement d'affections inflammatoires chez un mammifère.
